(19) Europäisches Patentamt
European Patent Office
Office européen des brevets

(11) EP 3 351 526 B1

(12) EUROPÄISCHE PATENTSCHRIFT

(45) Veröffentlichungstag und Bekanntmachung des Hinweises auf die Patenterteilung:
**18.11.2020 Patentblatt 2020/47**

(51) Int Cl.:
***C07C 69/82*** (2006.01) ***C08K 5/12*** (2006.01)
***C07C 67/03*** (2006.01)

(21) Anmeldenummer: **17152394.7**

(22) Anmeldetag: **20.01.2017**

# (54) DIISOPENTYLTEREPHTHALAT

DIISOPENTYLTEREPHTHALATE

TÉRÉPHTALATE DE DIISOPENTYLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**25.07.2018 Patentblatt 2018/30**

(73) Patentinhaber: **Evonik Operations GmbH**
**45128 Essen (DE)**

(72) Erfinder:
- **BOECK, Florian**
  **48143 Münster (DE)**
- **GRASS, Michael**
  **45721 Haltern am See (DE)**
- **WOLDT, Benjamin**
  **44892 Bochum (DE)**
- **HUBER, André**
  **45772 Marl (DE)**
- **BLEX, Christine**
  **45770 Marl (DE)**
- **BLUMENTHAL, Ulrike**
  **45770 Marl (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
**EP-A1- 3 059 221 EP-A1- 3 059 222**
**EP-A1- 3 059 223**

Bemerkungen:
Die Akte enthält technische Angaben, die nach dem Eingang der Anmeldung eingereicht wurden und die nicht in dieser Patentschrift enthalten sind.

EP 3 351 526 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft Di*i*sopentylterephthalat, dessen Herstellung, Weichmachergemische, Plastisole und Verkaufsprodukte umfassend Di*i*sopentylterephthalat sowie die Verwendung von Di*i*sopentylterephthalat als Weichmacher sowie zur Viskositätserniedrigung.

Im Bereich der Weichmacher für Polymere werden Terephthalsäureester als Ersatz oder Ergänzung zu Phthalsäureestern bereits seit einigen Jahren eingesetzt. Der kommerziell wichtigste Terephthalsäureester ist dabei Diethylhexylterephthalat, welches vereinfacht oft auch als Dioctylterephthalat bezeichnet wird. Terephthalsäureester, welche Alkoholreste mit weniger oder mehr als 8 Kohlenstoffatomen enthalten, werden im Stand der Technik ebenfalls beschrieben. Die Alkoholreste der Ester werden im Rahmen dieses Textes auch als Alkylreste (der Ester) bezeichnet.

Die Terephthalsäureester haben - unter anderem abhängig von der Kohlenstoffanzahl in den Alkoholresten der Esterfunktionen - unterschiedliche Eigenschaften und eignen sich dementsprechend mehr oder weniger stark für unterschiedliche Weichmacheranwendungen. So gelieren kürzerkettige Terephthalsäureester tendenziell bei niedrigeren Temperaturen als ihre längerkettigen Homologen. Eine niedrige Geliertemperatur eines Weichmachers stellt eine positive Eigenschaft insbesondere bei der Plastisolverarbeitung dar, da diese bei niedrigeren Temperaturen durchgeführt werden kann und zudem höhere Durchsätze bei der Verarbeitung erreicht werden können als bei der Verarbeitung von Plastisolen, welche Weichmacher mit einer hohen Geliertemperatur enthalten.

Gleichzeitig weisen Terephthalsäureester mit geringem Molekulargewicht und dementsprechend geringer Kohlenstoffanzahl im Alkoholrest jedoch eine höhere Flüchtigkeit auf als ihre schwereren Homologen. Eine hohe Flüchtigkeit eines Weichmachers ist ein gravierender Nachteil, da bei Austritt des Weichmachers nicht nur die Eigenschaften des weichgemachten Polymers verändert werden und somit die Langlebigkeit des Produktes verringert wird, sondern auch Weichmacher in die Umgebung freigesetzt wird.

Die Freisetzung von Weichmacher ist problematisch, da beispielsweise auf den Gebieten der Innenraumanwendungen, medizinischen Produkte, Spielzeuge, Kabel und im Automobilsektor zur Kommerzialisierung der Produkte Normen erfüllt werden müssen, welche die maximale Menge an aus einem Produkt austretenden organischen Verbindungen regeln, um die für Verbraucher und Umwelt erforderliche Sicherheit zu gewährleisten. So regelt beispielsweise der Ausschuss zur gesundheitlichen Bewertung von Bauprodukten (AgBB) in Übereinstimmung mit der vom Europäischen Parlament verabschiedeten Bauprodukte-Verordnung (Nr. 305/2011) die Vermeidung und Begrenzung von Schadstoffen in Innenräumen. Demnach sind Bauprodukte und damit auch Weichmacher-haltige Produkte nur dann für die Verwendung in Innenräumen von Gebäuden aus gesundheitlicher Sicht geeignet, wenn bestimmte Grenzwerte für emittierte VOC (flüchtige organische Verbindungen) und SVOC (schwer flüchtige organische Verbindungen) in einem genormten Messverfahren nicht überschritten werden. In Anlehnung an die DIN ISO 16000-6 werden als SVOC solche organische Verbindungen eingestuft, welche auf einer unpolaren Säule im Retentionsbereich von größer n-C16-Paraffin bis zu n-C22-Paraffin liegen (AgBB - Bewertungsschema für VOC aus Bauprodukten, Stand 2015). Produkte, welche höhere Emissionen als zugelassen aufweisen, können nur dann eingesetzt werden, wenn zusätzliche Maßnahmen, wie z.B. das Aufbringen einer Emissions-Sperrschicht aus Lack, die Überschreitung der maximal erlaubten Emissionsmenge verhindern. Die Notwendigkeit solcher zusätzlichen Maßnahmen verringert jedoch die Freiräume bei der Konfektionierung der Weichmacher in Produkten und verteuert somit die jeweilige Anwendung von Weichmachern, welche als VOC oder SVOC einzustufen sind. Zudem können sich durch die Notwendigkeit solcher zusätzlicher Schutzschichten weitere Schwierigkeiten ergeben wie beispielsweise eine erhöhte Anfälligkeit eines durch einen Lack geschützten SVOC-haltigen Produktes für Kratzer oder Abplatzungen.

Das Patent EP 1 808 457 B1 offenbart, dass Terephthalsäureester mit 4 bis 5 Kohlenstoffatomen in der längsten Kohlenstoffkette des Alkoholrestes als schnell gelierende Weichmacher gut geeignet sind. Dibutylterephthalate sind jedoch als SVOC einzustufen und ihr Einsatz ist deshalb mit den oben beschriebenen Nachteilen verknüpft. Zudem hängen einige anwendungsrelevante Eigenschaften von Dibutylterephthalten in signifikantem Maße von der Isomerenverteilung der Butylreste ab und die Viskosität von Dibutylterephthalat-haltigen Pasten erhöht sich bei Lagerung stark. Beide Eigenschaften wirken sich nachteilig bei der Anwendung aus.

Die Anmeldung WO 2010/071717 A1 beschreibt Terephthalsäurediester von $C_5$-$C_7$-Alkoholen und fokussiert sich deutlich auf Diheptylterephthalat. Diheptylterephthalat geliert im Vergleich zu Dipentylterephthalat jedoch bei deutlich höheren Temperaturen und ist deshalb als Schnellgelierer weniger gut geeignet als Dipentylterephthalat.

Anwendungsrelevante Eigenschaften der Weichmacher sind nicht nur von der Kohlenstoffanzahl in den Alkoholresten der Esterfunktionen abhängig, sondern auch von dem Verzweigungsgrad dieser Alkoholreste. So erläutert das Fachbuch Plasticisers, Principles and Practice, Alan S. Wilson, The Institute of Materials 1995, anhand der Phthalate, dass Weichmacher dann besonders vorteilhafte Eigenschaften, insbesondere eine geringe Viskosität und eine geringere Plastisolviskosität aufweisen, wenn die Alkoholreste der Esterfunktionen im Durchschnitt einen geringen Verzweigungsgrad aufweisen. In Übereinstimmung damit stellt das Dokument EP 1 808 457 B1 Terephthalsäureester als vorteilhaft heraus, wenn deren Alkylreste zum überwiegenden Teil lineare Pentylreste sind. Der Fachmann überträgt diese Erkenntnisse über vorteilhafte Isomerenverteilungen von Phthalaten und Terephthalaten auf andere Weichmacher, welche

ebenfalls Esterfunktionen enthalten.

Als Folge davon ist die Nachfrage nach linearen Alkoholen zur Weichmacherherstellung groß. Da ihr Anteil an den Hydroformylierungprodukten jedoch nur in Grenzen steuerbar ist, ist der Preis an Hydroformylierungsprodukten, welche einen hohen Anteil linearer Alkohole enthalten, in der Regel hoch, während gleichzeitig nach Absatzmöglichkeiten für verzweigte Hydroformylierungsprodukte, sprich für verzweigte Weichmacheralkohole, gesucht wird.

Die oben bereits als vorteilhaft bezeichnete geringe Viskosität von Weichmachern und die geringe Plastisolviskosität der aus den Weichmachern hergestellten Plastisole sind von hoher anwendungstechnischer Bedeutung, da Flüssigkeiten mit gewöhnlichen Pumpen lediglich bis zu einer Viskosität von zirka 1000 Pa·s zuverlässig gepumpt werden können. Weist ein Weichmacher oder ein Plastisol eine Viskosität oberhalb dieser Grenze auf, ist ein Transport lediglich mit Hilfe teurer Spezialpumpen oder bei erhöhter, die Viskosität des zu pumpenden Mediums verringernder Temperatur möglich.

Der vorliegenden Erfindung lag nun die Aufgabe zugrunde, einige, vorzugsweise alle der oben genannten Nachteile des Standes der Technik zu überwinden.

Es sollte vorzugsweise ein Weichmacher bereitgestellt werden, welcher zur Gewährleistung möglichst großer Freiräume bei der Formulierung nicht unter die Definitionen der nach deutschen oder internationalen Richtlinien reglementierten Verbindungen fällt. Dabei sollte mit Vorzug ein Weichmacher auf Basis von Terephthalsäureestern bereitgestellt werden.

Der Weichmacher sollte wirtschaftlich interessant sein, d.h. eine hohe Rohstoffausnutzung ermöglichen und gleichzeitig vorzugsweise mit möglichst geringem apparativem Aufwand verarbeitbar sein.

Die Aufgabe wird gelöst durch ein Di*i*sopentylterephthalat (DTP), dessen Pentylreste zu mehr als 25 Mol-% und gleichzeitig zu weniger als 60 Mol-% n-Pentylreste sind, wobei mindestens 60 Mol-% der im Diisopentylterephthalat gebundenen verzweigten isomeren Pentylreste 2-Methylbutylreste sind.

In anderen Worten werden die Vorteile durch ein Gemisch von Dipentylterephthalaten mit isomeren verzweigten und unverzweigten Pentylresten erreicht, in welchem zu mehr als 25 Mol-% und gleichzeitig zu weniger als 60 Mol-% der Pentylreste lineare Pentylreste (*n*-Pentylreste) sind. Dieses Gemisch wird im Folgenden als erfindungsgemäßes Di*i*sopentylterephthalat, erfindungsgemäßes (Pentyl-)Estergemisch oder auch kurz als DPT bezeichnet.

Im Rahmen dieses Textes wird vereinfacht oftmals von Alkylresten gesprochen, wenn die aus dem Alkohol stammenden Reste der Ester diskutiert werden. So wird vereinfacht der aus einem Pentanol stammende Rest einer Esterfunktion als Pentylrest bezeichnet.

Überraschend wurde gefunden, dass Plastisole, welche erfindungsgemäßes DPT enthalten, eine niedrige Plastisolviskosität aufweisen, welche zudem mit der Zeit nur in geringem Maße zunimmt. Plastisole enthaltend erfindungsgemäßes DPT sind demnach besonders lagerstabil. Es wurde gefunden, dass beide Eigenschaften - die geringe Plastisolviskosität sowie die gute Lagerbeständigkeit - zum einen innerhalb des beanspruchten Bereiches der Isomerenzusammensetzung des DPT außergewöhnlich vorteilhaft sind und zum anderen innerhalb dieses Bereiches überraschend wenig variieren und somit von der Isomerenverteilung nahezu unabhängig sind. Selbiges gilt für die Geliertemperatur der Plastisole. Somit weist erfindungsgemäßes DPT für den mit dessen Anwendung betrauten Fachmann auch bei Variation der Isomerenverteilung und selbst bei gleichzeitiger Unkenntnis derselben, stets vorteilhafte, verlässlich vorhersagbare Eigenschaften auf.

Des Weiteren wurde gefunden, dass das erfindungsgemäße DPT selbst ebenfalls eine geringe Viskosität aufweist und sich seine Viskosität erst bei überraschend niedrigen Temperaturen signifikant erhöht. Dies ermöglicht die Verarbeitung von erfindungsgemäßem DPT mit geringem apparativem Aufwand, da das DPT auch bei niedrigen oder schwankenden Temperaturen mittels normaler Pumpen gefördert werden kann und ohne dass zur Gewährleistung der Pumpbarkeit des DPT beheizte Rohrleitungen und Tanks oder spezielle Pumpen notwendig wären.

Vor dem Hintergrund der in der Literatur gelehrten angeblichen Überlegenheit linearer Alkylreste in Weichmachern überraschend, ist demnach DPT, welches erfindungsgemäß einen hohen Anteil an verzweigten Pentylresten aufweist, vorteilhaft einsatzbar. Dies ermöglicht die ökonomisch und ökologisch vorteilhafte Nutzung von Hydroformylierungsprodukten, welche einen hohen Anteil an verzweigten Pentanolen enthalten.

Das erfindungsgemäße Gemisch von Dipentylterephthalaten mit isomeren verzweigten und unverzweigten Pentylresten, in welchem weniger als 60 Mol-% der Pentylreste lineare Pentylreste (*n*-Pentylreste) sind, ist gemäß dem üblichen, oben beschriebenen Test weder als VOC noch als SVOC einzustufen, da keine der enthaltenen Gemischkomponenten im Retentionsbereich kleiner gleich n-C22-Paraffin liegt. Folglich ist sein Einsatz - im Gegensatz zum Einsatz von Gemischen isomerer Dibutylterephthalate - nicht durch deutsche oder internationale Richtlinien reglementiert.

In einem bevorzugten Di*i*sopentylterephthalat sind mehr als 27,5 Mol-% und insbesondere mehr als 30 Mol-% oder mehr als 35 Mol-% der Pentylreste im Estergemisch *n*-Pentylreste.

Vorteilhaft ist, dass die verzweigten isomeren Pentylreste im Estergemisch einen großen Anteil an 2-Methylbutylresten aufweisen. In einer bevorzugten Ausführungsform sind mindestens 70 Mol-%, weiter bevorzugt mindestens 80 Mol-%, besonders bevorzugt mindestens 90 Mol-% und insbesondere mindestens 95 Mol-% der im Estergemisch

gebundenen verzweigten isomeren Pentylreste 2-Methylbutylreste. Das erfindungsgemäße DPT enthält vorzugsweise bis 85 Mol-% und insbesondere bis 75 Mol-% 2-Methylbutylreste, bezogen auf alle enthaltenen Pentylreste.

In einer besonders bevorzugten Ausführungsform besteht das erfindungsgemäße DPT zu mindestens 75 Mol-%, weiter bevorzugt zu mindestens 90 Mol-% und insbesondere zu mindestens 95 Mol-% aus Estern, welche - vorzugsweise ausschließlich - 2-Methylbutyl- und/oder lineare Pentylreste enthalten, wobei das Molverhältnis von 2-Methylbutylresten zu linearen Pentylresten innerhalb dieses Estergemisches vorzugsweise im Bereich von 95:5 bis 40:60, insbesondere im Bereich von 70:30 bis 40:60 liegt.

Eine besonders niedrige Viskosität im Plastisol wird erreicht, wenn die verzweigten isomeren Pentylreste im Estergemisch zu einem signifikanten bis großen Anteil aus 3-Methylbutylresten bestehen. In einem solchen Fall sind mindestens 10 Mol-%, vorzugsweise mindestens 20 Mol-%, bevorzugt mindestens 30 Mol-%, weiter bevorzugt mindestens 40 Mol-%, mit Vorzug dazu mindestens 50 Mol-%, vorzugsweise mindestens 60 Mol-%, weiter bevorzugt mindestens 70 Mol-%, besonders bevorzugt mindestens 80 Mol-% und insbesondere mindestens 90 Mol-% der im Estergemisch gebundenen verzweigten isomeren Pentylreste 3-Methylbutylreste. Es kann des Weiteren von Vorteil sein, wenn das erfindungsgemäße DPT zu mindestens 75 Mol-% und insbesondere zu mindestens 90 Mol-% aus Estern besteht, welche - vorzugsweise ausschließlich - 3-Methylbutyl- und/oder lineare Pentylreste enthalten, und dabei das Molverhältnis von 3-Methylbutylresten zu linearen Pentylresten im Bereich von 95:5 bis 40:60, insbesondere im Bereich von 70:30 bis 40:60 liegt.

Wie bereits beschrieben, weist erfindungsgemäßes DPT bis zu niedrigen Temperaturen eine geringe Viskosität auf und ist damit über einen breiten Temperaturbereich problemlos und ohne weiteren Aufwand pumpbar. Bevorzugtes DPT weist bei Temperaturen oberhalb von 10 °C eine Viskosität kleiner 1000 Pa·s, bevorzugt kleiner 500 Pa·s auf. Die Viskosität liegt vorteilhafterweise bei Temperaturen oberhalb von 5 °C, vorzugsweise bei Temperaturen oberhalb von 0 °C und insbesondere bei Temperaturen oberhalb von -5 °C bei Werten kleiner 1000 Pa·s. Die Viskosität wird vorzugsweise mit einem Rheometer mittels oszillierendem Platte-Platte-System, bevorzugt mit einer Messspaltbreite von 0,5 mm bestimmt. Mit besonderem Vorzug wird die Viskosität gemessen, wie im experimentellen Teil, Beispiel 9 beschrieben.

Überraschend wurde gefunden, dass erfindungsgemäße Gemische isomerer Dipentylterephthalate (erfindungsgemäßes DPT) zum überwiegenden Teil zwar gemäß DSC-Messung (Dynamische Differenzkalorimetrie) Schmelzpunkte im Bereich unterhalb von -10 °C (onset) aufweisen, dennoch bei diesen und noch niedrigeren Temperaturen eine genügende niedrige Viskosität aufweisen um noch mit normalen Pumpen förderbar zu sein. Es wurde gefunden, dass erfindungsgemäßes DPT, welches einen Boeck-Faktor kleiner 100, vorzugsweise kleiner 90, weiter bevorzugt kleiner 70, besonders bevorzugt kleiner 50 und insbesondere kleiner 30 oder sogar kleiner 10 aufweist, auch bei tiefen Temperaturen mit geringem apparativen und energetischen Aufwand pumpbar ist. Die Bestimmung des Boeck-Faktors wird im experimentellen Teil erläutert. Vorzugsweise liegt der Boeck-Faktor des erfindungsgemäßen DPT bei Werten kleiner 100, bevorzugt kleiner 90, vorzugsweise kleiner 50, insbesondere kleiner 10.

Der Effekt der niedrigen Viskosität selbst bei geringen Temperaturen tritt bevorzugt auch im Gemisch des erfindungsgemäßen DPTs mit einem oder mehreren Weichmachern, insbesondere im Gemisch mit mindestens einem Primärweichmacher auf. Weiterer Gegenstand der vorliegenden Erfindung ist deshalb ein Gemisch umfassend erfindungsgemäßes DPT und mindestens einen zusätzlichen Weichmacher.

Als zusätzlicher Weichmacher kommen Alkylbenzoate, Dialkyladipate, Glycerinester, Citronensäuretrialkylester, acylierte Citronensäuretrialkylester, Trialkyltrimellitate, Glykoldibenzoate, andere Dialkylterephthalate, Ester der Furandicarbonsäure, Dialkanoylester von Dianhydrohexitolen (z.B. Isosorbit) und Dialkylester der 1,2-, 1,3- oder 1,4-Cyclohexandicarbonsäure in Betracht. In einer besonders bevorzugten Ausführungsform enthält die Mischung der Weichmacher weniger als 5 Massen-% und insbesondere weniger als 0,5 Massen-% Phthalat-haltige Verbindungen. In einer weiteren bevorzugten Ausführungsform ist der zusätzliche Weichmacher kein Diheptylterephthalat.

Das Mengenverhältnis von erfindungsgemäßen DPT zu zusätzlichem Weichmacher liegt vorzugsweise bei 80:20 bis 3:97, bevorzugt bei 60:40 bis 10:90, besonders bevorzugt bei 50:50 bis 20:80 und insbesondere bei 40:60 bis 25:75.

Eine bevorzugte Kombination ist erfindungsgemäßes DPT im Gemisch mit einem oder mehreren Estern der Cyclohexandicarbonsäure, insbesondere mit den 1,2-, 1,3- oder 1,4-Estern, deren Alkyl- oder Alkoholreste der Esterfunktionen 8 bis 10 Kohlenstoffatome enthalten. Besonders bevorzugt wird erfindungsgemäßes DPT im Gemisch mit Cyclohexan-1,2-dicarbonsäuredi*i*sononylester oder Cyclohexan-1,4-dicarbonsäuredi*i*sononylester eingesetzt.

Günstig sind auch Kombinationen von erfindungsgemäßem DPT mit Terephthalaten, welche 8 bis 10 Kohlenstoffatome in dem Alkyl- oder Alkoholrest der Esterfunktion enthalten, insbesondere mit Di*i*sononylterephthalat oder Diethylhexylterephthalat. Von Vorteil sind ebenfalls Kombinationen von DPT mit Furanoaten, deren Alkylgruppen der Esterfunktionen 8 bis 10 Kohlenstoffatome umfassen, Kombinationen von DPT mit Alkylsulfonsäureester des Phenols oder Polyolestern wie beispielsweise Pentaerythrit-Tetravalerat.

In einer Ausführungsform wird erfindungsgemäßes DPT mit $C_8$-$C_{10}$-Phthalaten, insbesondere $C_9$- oder $C_{10}$-Phthalaten kombiniert. Besonders bevorzugt sind dabei Kombinationen von erfindungsgemäßem DPT mit DINP (Di*i*sononylphthalat), DIDP (Di*i*sodecylphthalat) und/oder DPHP (Dipropylheptylphthalat). Bevorzugt enthalten diese

Weichmachergemische weniger als 5 Massen-% und insbesondere weniger als 0,5 Massen-% andere Phthalat-haltige Verbindungen.

Weiterer Gegenstand der vorliegenden Erfindung ist ein Plastisol umfassend erfindungsgemäßes Di*i*sopentylterephthalat. Dieses Plastisol weist die oben bereits beschrieben Vorteile, insbesondere eine geringe Plastisolviskosität und eine gute Lagerfähigkeit auf.

Das Plastisol enthält vorzugsweise ein Polymer oder mehrere Polymere. Geeignete Polymere sind vorzugsweise ausgewählt aus der Gruppe, die gebildet wird durch Polyvinylchlorid (PVC), Homo- oder Copolymere auf Basis von Ethylen, Propylen, Butadien, Vinylacetat, Glycidylacrylat, Glycidylmethacrylat, Ethylacrylat, Butylacrylat oder Methacrylat mit Alkoxyresten von verzweigten oder unverzweigten Alkoholen mit einem bis zehn Kohlenstoffatom(en), Acrylnitril oder cyclischen Olefinen, Polyvinylidenchlorid (PVDC), Polyacrylaten, insbesondere Polymethylmethacrylat (PMMA), Polyalkylmethacrylat (PAMA), Polyharnstoffe, silylierte Polymere, Fluorpolymere, insbesondere Polyvinylidenfluorid (PVDF), Polytetrafluorethylen (PTFE), Polyvinylacetat (PVAc), Polyvinylalkohol (PVA), Polyvinylacetale, insbesondere Polyvinylbutyral (PVB), Polystyrolpolymere, insbesondere Polystyrol (PS), expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi und Silikone.

Bevorzugte Polymere sind Polyvinylchlorid, Co-Polymere von Vinylchlorid mit Vinylacetat oder mit Butylacrylat, Polyalkylmethacrylat (PAMA), Polyvinylbutyral (PVB), Polyurethan, Polysulfide, Polymilchsäure (PLA), Polyhydroxybutyral (PHB) und Nitrocellulose. Besonders bevorzugt ist PVC. Ganz besonders bevorzugt sind Emulsions- oder Mikrosuspensions-PVC.

In bevorzugter Weise beträgt die Menge an erfindungsgemäßem DPT im Plastisol 5 bis 120 Massenteile, bevorzugt 10 bis 100 Massenteile, besonders bevorzugt 15 bis 90 Massenteile und ganz besonders bevorzugt 20 bis 80 Massenteile pro 100 Massenteile Polymer.

In einer Ausführungsform wird das Plastisol zu einem Schaum verarbeitet.

Es ist dabei bevorzugt, dass das Plastisol einen Schaumbildner enthält. Dieser Schaumbildner kann eine Gasblasen-entwickelnde Verbindung, die optional zusammen mit einem sog. "Kicker" verwendet wird, sein. Als solche Kicker werden in der Regel metallhaltige Verbindungen bezeichnet, die den thermischen Zerfall der Gasblasen entwickelnden Komponente katalysieren und dazu führen, dass der Schaumbildner unter Gasentwicklung reagiert und das Plastisol aufgeschäumt wird. Schaumbildner werden auch als Treibmittel bezeichnet. Grundsätzlich kann das Plastisol chemisch (d.h. mittels Treibmittel) oder mechanisch (d.h. durch Einarbeitung von Gasen, bevorzugt von Luft) aufgeschäumt werden. Als Gasblasen entwickelnde Komponente (Treibmittel) wird vorzugsweise eine Verbindung verwendet, die unter Wärmeeinfluss in gasförmige Bestandteile zerfällt, und damit ein Aufblähen des Plastisols bewirkt.

Die für die Herstellung von Polymerschäumen geeigneten Treibmittel zum Aufschäumen schließen alle Typen bekannter Treibmittel, physikalische und/oder chemische Treibmittel einschließlich anorganischer Treibmittel und organischer Treibmittel, ein.

Beispiele für chemische Treibmittel sind Azodicarbonamid, Azodiisobutyronitril, Benzolsulfonylhydrazid, 4,4-Oxybenzolsulfonylsemicarbazid, 4,4-Oxybis(benzolsulfonylhydrazid), Diphenylsulfon-3,3-disulfonylhydrazid, p-Toluolsulfonylsemicarbazid, N,N-Dimethyl-N,N-dinitrosoterephthalamid und Trihydrazintriazin, N=N-Dinitrosopentamethylentetramin, Dinitrosotrimethyltriamin, Natriumhydrogencarbonat, Natriumbicarbonat, Mischungen von Natriumbicarbonat und Zitronensäure, Ammoniumcarbonat, Ammoniumbicarbonat, Kaliumbicarbonat, Diazoaminobenzol, Diazoaminotoluol, Hydrazodicarbonamid, Diazoisobutyronitril, Bariumazodicarboxylat und 5-Hydroxytetrazol. Besonders bevorzugt handelt es sich bei mindestens einem der eingesetzten Treibmittel um Azodicarbonamid, welches bei Reaktion gasförmige Komponenten wie $N_2$, $CO_2$ und CO freisetzt. Die Zerfallstemperatur des Treibmittels kann durch den Kicker verringert werden.

Mechanisch geschäumte Zusammensetzungen werden auch als "Schlagschaum" bezeichnet.

Alternativ zur Verarbeitung des Plastisols zu einem Schaum kann dieses auch nichtaufgeschäumt (also kompakt) zum Beispiel zu einer Folie oder einer Beschichtung weiterverarbeitet werden. Bevorzugt ist die Verarbeitung eines oder mehrerer unterschiedlicher Plastisole zu mehrschichtigen Systemen, in denen eine oder mehrere Schichten aus geschäumtem und eine oder mehrere Schichten aus nicht-geschäumtem Plastisol hergestellt wurden. Denkbar sind ebenfalls mehrschichtige System, welche ausschließlich aus geschäumtem oder alternativ ausschließlich aus nicht-geschäumtem Plastisolem hergestellt wurden. Hierbei kann es bevorzugt sein, dass nur eine der Schichten erfindungsgemäßes DPT enthält oder dass zwei oder mehr Schichten eines entsprechend mehrschichtigen Systemes erfindungsgemäßes DPT - optional im Gemisch mit einem oder mehreren anderen weichmachenden Verbindungen - enthält.

Beispiele für mehrschichtige Systeme sind Kunstleder oder CV-Bodenbeläge (CV = cushion vinyl). Des Weiteren können Plastisole zu Handschuhen, zu Spielzeugen wie beispielsweise Puppenköpfen (über Rotationsverfahren) oder auch zu Unterbodenschutz (durch Applikations des Plastisols auf die Fahrzeugunterseite) verarbeitet werden.

Unabhängig von der Weiterverarbeitungsart kann das Plastisol Additive enthalten, die insbesondere ausgewählt sind aus der Gruppe bestehend aus Füllstoffen/Verstärkungsmitteln, Pigmenten, Mattierungsmitteln, Thermostabilisatoren, CoStabilisatoren mit weichmachender Wirkung, Antioxidantien, UV-Stabilisatoren, Costabilisatoren, Lösungsmitteln, Viskositätsregulierern, Schaumstabilisatoren, Flammschutzmitteln, Haftvermittlern und Verarbeitungs- bzw. Prozesshilfsstoffen (wie z.B. Gleitmitteln).

Wie bereits beschrieben ist erfindungsgemäßes DPT besonders geeignet, die Viskosität innerhalb von Weichmachergemischen und Plastisolen zu senken. Zudem zeichnen sich die DPT-haltigen Mischungen inklusive der resultierenden Plastisole durch eine verbesserte Lagerfähigkeit aus. Ein weiterer Gegenstand der vorliegenden Erfindung ist deshalb die Verwendung von erfindungsgemäßem DPT zur Viskositätserniedrigung und/oder zur Verbesserung der Lagerfähigkeit von Weichmachergemischen oder Plastisolen.

Die geringe Viskosität des erfindungsgemäßen DPTs auch bei niedrigen Temperaturen (beispielsweise bei -40 °C) ist besonders in solchen Regionen von Vorteil, in denen klimabedingt außerhalb von Gebäuden und damit auch in nicht-geheizten Industrieanlagen, Temperaturen vorherrschen, bei denen viele Einsatzstoffe hochviskos oder sogar fest sind. Gegenstand der vorliegenden Erfindung ist demnach die Verwendung von erfindungsgemäßem DPT bei der Herstellung von Plastisolen bei Umgebungstemperaturen, welche nicht verlässlich oberhalb von 20 °C liegen. In bevorzugten Ausführungsformen wird das erfindungsgemäße DPT bei der Herstellung von Plastisolen bei Umgebungstemperaturen verwendet, welche nicht verlässlich oberhalb von 15 °C, 10 °C, 5 °C, 0 °C, -5°C oder sogar nicht verlässlich oberhalb von -10°C liegen. Im Rahmen der vorliegenden Erfindung liegt eine Temperatur nicht verlässlich über einem gewissen Wert, wenn die Temperatur auch nur 1x im Monat oder auch nur 1x innerhalb eines Jahres den angegebenen Wert unterschreitet. Dementsprechend liegt eine Temperatur dann verlässlich oberhalb eines bestimmenten Wertes, wenn die Temperatur stets höher ist als der angegebene Wert.

Die Verwendung des erfindungsgemäßen DPTs ermöglicht die Einsparung aufwendiger Heiz- und Isoliersysteme sowie den Verzicht auf den Einsatz spezieller Pumpen für hochviskose Medien, da erfindungsgemäßes DPT sowie die daraus hergestellten Weichmachergemische auch bei tiefen Temperaturen aufgrund ihrer niedrigen Viskosität problemlos pumpbar sind.

Das erfindungsgemäße DPT, ein dieses DPT umfassende Weichmachergemisch oder ein Plastisol umfassend das erfindungsgemäße DPT wird vorzugsweise zu Unterbodenschutzmassen für Fahrzeuge, Tapeten, Gewebebeschichtungen, Kunstleder oder Fußbodenbelägen, insbesondere elastischen Fußbodenbelägen, weiterverarbeitet. Gegenstand der vorliegenden Erfindung ist ein Produkt, insbesondere ein Unterbodenschutz, eine Tapete, eine Gewebebeschichtung, ein Kunstleder oder ein Fußbodenbelag, welches erfindungsgemäßes DPT enthält. Das Produkt kann auch alternativ ein Gemisch aus erfindungsgemäßem DPT und mindestens einen weiteren Weichmacher oder ein Plastisol umfassend erfindungsgemäßes DPT enthalten.

Unabhängig davon, ob es aus einem Plastisol hergestellt wurde, kann das Produkt ein Polymer oder mehrere Polymere enthalten. Geeignete Polymere sind vorzugsweise ausgewählt aus der Gruppe, die gebildet wird durch Polyvinylchlorid (PVC), Homo- oder Copolymere auf Basis von Ethylen, Propylen, Butadien, Vinylacetat, Glycidylacrylat, Glycidylmethacrylat, Ethylacrylat, Butylacrylat oder Methacrylat mit Alkoxyresten von verzweigten oder unverzweigten Alkoholen mit einem bis zehn Kohlenstoffatom(en), Acrylnitril oder cyclischen Olefinen, Polyvinylidenchlorid (PVDC), Polyacrylaten, insbesondere Polymethylmethacrylat (PMMA), Polyalkylmethacrylat (PAMA), Polyharnstoffe, silylierte Polymere, Fluorpolymere, insbesondere Polyvinylidenfluorid (PVDF), Polytetrafluorethylen (PTFE), Polyvinylacetat (PVAc), Polyvinylalkohol (PVA), Polyvinylacetale, insbesondere Polyvinylbutyral (PVB), Polystyrolpolymere, insbesondere Polystyrol (PS), expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), CelluloseAcetat/Butyrat (CAB), Gummi und Silikone.

Bevorzugte Polymere sind Polyvinylchlorid, Co-Polymere von Vinylchlorid mit Vinylacetat oder mit Butylacrylat, Polyalkylmethacrylat (PAMA), Polyvinylbutyral (PVB), Polyurethan, Polysulfide, Polymilchsäure (PLA), Polyhydroxybutyral (PHB) und Nitrocellulose. Besonders bevorzugt ist PVC. Ganz besonders bevorzugt sind Emulsions- oder Mikrosuspensions-PVC.

Ein weiterer Gegenstand der vorliegenden Erfindung ist die Verwendung von erfindungsgemäßem DPT als Weichmacher in Kunststoffzusammensetzungen, insbesondere in PVC-haltigen Kunststoffzusammensetzungen.

Das erfindungsgemäße DPT wird vorzugsweise als Weichmacher in Klebstoffen, Dichtungsmassen, Beschichtungsmassen, Lacken, Farben, Plastisolen, Schäumen, Kunstleder, Fußbodenbelägen (z.B. Deckschicht), Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, beispielsweise Schläuchen oder Blutbeuteln, verwendet.

Erfindungsgemäßes DPT findet vorzugsweise als Schnellgelierer Verwendung, welcher es ermöglicht, Plastisole bei besonders niedrigen und somit günstigen Verarbeitungstemperaturen herzustellen und weiterzuverarbeiten.

Bezogen auf 100 Massenteile Polymer enthalten bevorzugte Mittel von 5 bis 200, bevorzugt von 10 bis 150 Massenteile an Weichmacher.

Gegenstand der vorliegenden Erfindung ist zudem ein Verfahren zur Herstellung von erfindungsgemäßem DPT durch Veresterung von Terephthalsäure oder Umesterung eines Terephthalsäureesters mit einem Gemisch isomerer Pentanole.

Vorzugsweise werden in dem erfindungsgemäßen Verfahren weniger als 15 Gew.-%, bevorzugt weniger als 10 Gew.-%, weiter bevorzugt weniger als 5 Gew.-% und insbesondere weniger als 2 Gew.-% oder weniger als 1 Gew.-% an Alkoholen eingesetzt, welche mehr oder weniger als 5 Kohlenstoffatome enthalten, also keine Pentanole sind. Die Angabe in Gew.-% ist dabei auf die Summe aller in dem Verfahren eingesetzten Alkohole bezogen.

Das in dem erfindungsgemäßen Verfahren eingesetzte Pentanolgemisch enthält vorzugsweise weniger als 60 Mol-% *n*-Pentanol. Der Mindestgehalt an *n*-Pentanol im Gemisch der isomeren Pentanol liegt vorzugsweise bei mindestens 2 Mol-%, bevorzugt bei mindestens 10 Mol-%, weiter bevorzugt bei mehr als 20 Mol-%, mit Vorzug dazu bei mehr als 22,5 Mol-% oder sogar bei mehr als 25 Mol-%, weiter bevorzugt bei mehr als 27,5 Mol-%, 30 Mol-% oder sogar bei mehr als 35 Mol-%.

Die bevorzugten Mengenverhältnisse und Anteile von *n*-Pentanol, 2-Methylbutanol und 3-Methylbutanol in dem im erfindungsgemäßen Verfahren eingesetzten Gemisch isomerer Pentanole entspricht den Mengenverhältnissen und Anteilen, welche für die Alkylreste des erfindungsgemäßen DPTs im Vorfeld bereits beschrieben wurden. Zur Vermeidung von Wiederholungen sei auf die voranstehenden Textstellen verwiesen.

Werden die erfindungsgemäßen Gemische durch Umesterung hergestellt, so wird/werden vorzugsweise ein oder mehrere Terephthalsäureester, in dem/denen die Alkylreste der Esterfunktionen jeweils weniger als 4 Kohlenstoffatome umfassen, mit einem Gemisch isomerer Pentanole umgeestert.

Bevorzugt wird Terephthalsäuredimethylester oder Terephthalsäurediethylester, insbesondere Terephthalsäuredimethylester zu den erfindungsgemäßen Gemischen von isomeren Dipentylterephthalaten umgeestert.

Die Veresterung beziehungsweise Umesterung wird vorzugsweise in Anwesenheit eines Katalysators oder mehrerer Katalysatoren, beispielsweise unter Verwendung von Brönstedt- oder Lewis-Säuren oder -Basen als Katalysator, durchgeführt. Als besonders geeignete Katalysatoren haben sich Schwefelsäure, Methansulfonsäure, *p*-Toluolsulfonsäure und Metallverbindungen erwiesen. Beispiele für besonders bevorzugte Katalysatoren sind Zinnpulver, Zinn(II)oxid, Zinn(II)oxalat, Titankatalysatoren wie Tetra*iso*propylorthotitanat, Tetrabutylorthotitanat oder Tetrapentylorthotitanat sowie Zirkoniumester wie Tetrabutylzirkonat oder Tetrapentylzirkonat. Beispiele für besonders bevorzugte basische Katalysatoren sind Alkoholate wie Natriummethanolat und Kaliummethanolat.

Um das sich bei der Reaktion ausbildende Gleichgewicht zu Gunsten der erfindungsgemäßen Gemische zu verschieben, kann es vorteilhaft sein, das bei der Veresterung entstehende Wasser beziehungsweise den bei der Umesterung entstehenden Alkohol aus dem Reaktionsgemisch abzudestillieren. Vorzugsweise wird ein Azeotrop von Wasser und Alkohol abdestilliert. Aufgrund einer möglichen Schaumbildung kann hier mit einer Kolonne gearbeitet werden.

Zudem kann es vorteilhaft sein, das Gemisch isomerer Pentanole insgesamt im Überschuss einzusetzen. Vorzugsweise wird das Gemisch isomerer Pentanole in einem Überschuss von 5 bis 50 Mol-%, insbesondere 9 bis 30 Mol-% der zur Bildung des erfindungsgemäßen Gemisches von Dipentylterephthalten notwendigen molaren Menge eingesetzt. Mit Vorzug wird der nach Beendigung der Reaktion übrig bleibende Überschussalkohol für eine weitere Veresterung oder Umesterung oder eine andere chemische Reaktion wiederverwendet. Hierzu kann der Überschussalkohol zur Erhöhung seiner Reinheit aufgearbeitet werden. So ist es beispielsweise möglich, ein abdestilliertes Alkohol-Wasser-Azeotrop zumindest teilweise zu kondensieren, das Kondensat in eine wässrige und eine organische Phase aufzutrennen, aus der organischen Phase unerwünschte Nebenprodukte - wie beispielsweise durch Wasserabspaltung aus dem Alkohol gebildete Olefine - abzutrennen, bevor die dann aufgereinigte organische Phase wieder in das Reaktionssystem zurückführt wird oder für eine andere Reaktion oder zu einem anderen Zweck Verwendung findet.

Möglich ist es zudem, das Reaktionsgemisch der Veresterung beziehungsweise Umesterung mit überhitztem Alkoholdampf zu behandeln. Hierdurch kann ein Teil des Energieeintrages durch andere Medien eingespart sowie eine gute Durchmischung des Reaktionsmediums erreicht werden.

Andere Möglichkeiten der Energieeinsparung bestehen darin, das Gemisch isomerer Pentanole mit einer Temperatur oberhalb der Umgebungstemperatur, beispielsweise mit 40 °C oder 60 °C in das Reaktionssystem einzuspeisen. Auch Terephthalsäuredimethylester kann mit erhöhter Temperatur, vorzugsweise als Schmelze in dem erfindungsgemäßen Verfahren eingesetzt werden. Neben dem Vorteil des Energieeintrages ermöglicht diese Verfahrensweise zudem eine bessere Durchmischung des Reaktionsmediums und eine rascher ablaufende Reaktion.

Nach Beendigung der Veresterung oder Umesterung wird das jeweilige Reaktionsgemisch in üblicher Art und Weise aufgearbeitet. So ist es beispielsweise möglich, den rohen Ester bei einem erhöhten Druck, welcher mindestens so groß ist wie der Dampfdruck von Wasser bei der vorherrschenden Temperatur, mit einer wässrigen Base zu behandeln. Diese Verfahrensführung ermöglicht es, gut filtrierbare Reaktionsmischungen zu erhalten.

**Beispiele:**

Beispiele 1 - 8: Herstellung der Estergemische

In eine Apparatur umfassend Reaktionskolben mit Rührer, Thermometer, aufgesetzter 20 cm Raschigringkolonne mit Destillationskopf und Auffangkolben sowie Tauchrohr mit aufgesetzten Tropftrichter, wurden 485 g Dimethylterephthalat (2,5 Mol, Reinheit 99,9 %) und $m_{av}$ des Alkohols A und $m_{bv}$ des Alkohols B eingefüllt. Die Apparatur wurde eine Stunde mit Stickstoff (6 l/h) über das Tauchrohr gespült. Anschließend wurden 0,43 g Tetra-n-Butyltitanat ($1{,}25 \cdot 10^{-3}$ Mol, Sigma Aldrich, Reinheit > 97 %) hinzugegeben. Die Reaktion wurde unter Rühren bis auf Siedetemperatur aufgeheizt. Ab diesem Zeitpunkt fiel Methanol an, das kontinuierlich über den Destillationskopf aus der Reaktion entfernt wurde. Die Methanolabnahme erfolgte bei einer Kopftemperatur von 65 bis 68 °C. Oberhalb von 68 °C wurde kein Methanol aus dem System entfernt. Nachfolgend wurden weitere $m_{an}$ des Alkohols A und $m_{bn}$ des Alkohols B über den Tropftrichter und das Tauchrohr zudosiert, so dass die Reaktionstemperatur nicht unter 200 °C fiel. Im Verlauf der Umesterung fielen 160 g Methanol (5 Mol) an.

Nach vollständiger Alkoholzugabe wurden stündlich Proben aus der Reaktion genommen und mittels Gaschromatographie analysiert. Sobald mittels Gaschromatographie weniger als 0,5 Flächen-% Monomethylmischester detektiert wurden, wurde die Heizung abgeschaltet und langsam Vakumm angelegt (Endvakuum 1 mbar). Nach Erreichen des Endvakuums wurde langsam bis auf 160 °C aufgeheizt. Nach erfolgter Entfernung des Überschussalkohols wurde die Heizung abgestellt und die Reaktion unter Vakuum und Einleiten von Stickstoff auf 80 °C abgekühlt. Anschließend wurde bei dieser Temperatur die Säurezahl des Rohprodukts gemessen.

Das Rohprodukt wurde mit der 3-fachen stöchiometrischen Menge 10 %-iger Natronlauge (bezogen auf die theoretische Stoffmenge an Säure) versetzt und bei 80 °C für 15 Minuten unter Stickstoff gerührt. Anschließend wurde unter Vakuum auf 160 °C aufgeheizt und unter kontinuierlichem Einleiten von Stickstoff enthaltene Spuren an Leichtsieder entfernt. Hierbei wurden stündlich Proben entnommen und mittels Gaschromatographie analysiert. Nach Erreichen von weniger als 0,025 Flächen-% Restalkohol in der Probe laut Gaschromatographie, wurde das Produkt erneut auf 80 °C abgekühlt und über einen Büchnertrichter mit Filterpapier und vorgepresstem Filterkuchen aus Filterhilfsmittel (Perlite Typ D14) mittels Vakuum in eine Saugflasche filtriert. An dem Filtrat wurde wiederum eine gaschromatographische Analyse durchgeführt, anhand derer die Reinheit (R) und die Zusammensetzung des Produkts analysiert wurde.

Tabelle 1: Details der Synthesen und erhaltenen Estergemische (Beispiele 1 - 8)

| | Alkohol A = *n*-Pentanol | | Alkohol B = 2-Methylbutanol | | Reinheit R [%] |
|---|---|---|---|---|---|
| | $m_{av}$ [g] | $m_{an}$ [g] | $m_{bv}$ [g] | $m_{bn}$ [g] | |
| Beispiel 1 | 380,2 | 0 | 126,7 | 0 | 99,7 |
| Beispiel 2 | 283,8 | 0 | 223,0 | 0 | 99,9 |
| Beispiel 3 | 253,4 | 0 | 253,4 | 0 | 99,8 |
| Beispiel 4 | 177,4 | 0 | 329,5 | 0 | 99,6 |
| Beispiel 5 | 101,4 | 0 | 405,5 | 0 | 99,7 |
| | Alkohol A = *n*-Butanol | | Alkohol B = 2-Methylpropanol | | |
| | $m_{av}$ [g] | $m_{an}$ [g] | $m_{bv}$ [g] | $m_{bn}$ [g] | |
| Beispiel 6 | 278,0 | 41,7 | 92,7 | 13,9 | 99,7 |
| Beispiel 7 | 185,3 | 27,8 | 185,3 | 27,8 | 99,8 |
| Beispiel 8 | 92,7 | 13,9 | 278,0 | 41,7 | 99,1 |

2-Methylpropanol: Firma Oxea, Reinheit > 99,5 %
*n*-Butanol: Firma Sigma Aldrich, Reinheit > 99 %
2-Methylbutanol: Firma Sigma Aldrich, Reinheit > 99,9 %
*n*-Pentanol: Firma Sigma Aldrich, Reinheit > 99,9 %

Die Ermittlung der Zusammensetzung des Pentyl- bzw. des Butylestergemisches kann durch $^{1}H$-NMR und $^{13}C$-NMR erfolgen. Die NMR-spektroskopischen Untersuchungen können prinzipiell mit jedem handelsüblichen NMR-Gerät durchgeführt werden.

Im vorliegenden Fall wurde die Zusammensetzung der Gemische mittels $^{1}H$-NMR-Spektroskopie an einer Lösung der Ester in Deuterochloroform ($CDCl_3$) bestimmt. Für die Aufnahme der Spektren wurden 20 mg Substanz in 0,6 ml $CDCl_3$ (enthaltend 1 Massen-% TMS) gelöst und in ein NMR-Röhrchen mit einem Durchmesser von 5 mm gefüllt. Für die vorliegenden NMR-spektroskopischen Untersuchungen wurde ein Gerät vom Typ Avance 500 der Firma Bruker eingesetzt. Die Spektren wurden bei einer Temperatur von 300 K mit einem Delay (Verzögerung) von d1 = 5 Sekunden, 32 Scans (Durchgänge), einer Pulslänge von ca. 9,5 $\mu$s (90° Anregungspuls) und einer Sweep Width (Spektrale Breite) von 10000 Hz mit einem 5 mm BBO-Probenkopf (broad band observer; Breitbandbeobachtung) aufgenommen. Die Resonanzsignale werden gegen die chemische Verschiebung von Tetramethylsilan (TMS = 0 ppm) als interner Standard aufgezeichnet. Mit anderen handelsüblichen NMR-Geräten werden mit den gleichen Betriebsparametern vergleichbare Ergebnisse erhalten.

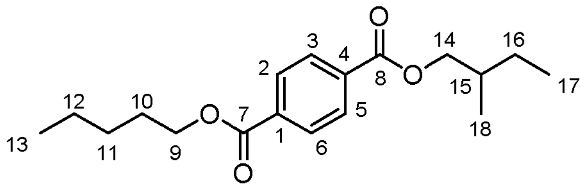

DPT

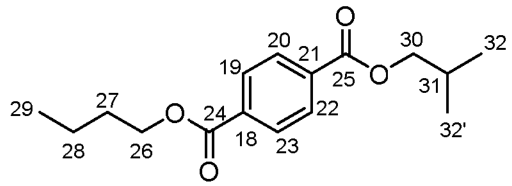

DBT

Die erhaltenen $^{1}H$-NMR-Spektren der Gemische weisen im Bereich von 4,0 bis 4,5 ppm Resonanzsignale auf, die durch die Signale der Wasserstoffatome der Methylgruppen in direkter Nachbarschaft zum Sauerstoff des Alkohols bzw. des Alkoholrests gebildet werden ($C^9H_2$ und $C^{14}H_2$; $C^{26}H_2$ und $C^{30}H_2$). Hierbei erfahren die Protonen an $C^9$ und $C^{26}$ eine stärkere Tieffeldverschieb (Triplett bei ca. 4,35 ppm) als die Protonen von $C^{14}$ und $C^{30}$ (Multiplett zwischen 4,10 und 4,25 ppm). Die Quantifizierung erfolgt durch vergleichende Bestimmung der Fläche unter den jeweiligen Resonanzsignalen, also der vom Signal von der Grundlinie eingeschlossenen Fläche. Handelsübliche NMR-Geräte verfügen über Vorrichtungen zur Integration der Signalfläche. In den vorliegenden NMR-spektroskopischen Untersuchungen wurde die Integration mit Hilfe der Software TopSpin®, Version 3.1 durchgeführt. Der Anteil der linearen Alkyreste innerhalb des jeweiligen Estergemisches kann mittels folgender Berechnung erschlossen werden.

$$\text{Mol-\% }(n\text{-Butylester}) = \frac{I\,(C^{26}H_2)}{I\,(C^{26}H_2) + I\,(C^{30}H_2)} \times 100$$

$$\text{Mol-\% }(n\text{-Pentylester}) = \frac{I\,(C^{9}H_2)}{I\,(C^{9}H_2) + I\,(C^{14}H_2)} \times 100$$

Tabelle 2: untersuchte Estergemische und der Anteil linearer Alkylreste in % gemäß NMR

| | Anteil *n*-Alkohol im Reaktionsgemisch | Anteil *n*-Alkylrest im Produkt | Bezeichnung |
|---|---|---|---|
| Beispiel 1 | 75 % | 77,4 % | DPT (77 % n) |
| Beispiel 2 | 56 % | 58,4 % | DPT (58 % n) |
| Beispiel 3 | 50 % | 52,9 % | DPT (53 % n) |
| Beispiel 4 | 35 % | 37,6 % | DPT (38 % n) |
| Beispiel 5 | 20 % | 20,6 % | DPT (21 % n) |
| Beispiel 6 | 75 % | 78,1 % | DBT (78 % n) |
| Beispiel 7 | 50 % | 54,0 % | DBT (54 % n) |
| Beispiel 8 | 25 % | 27,8 % | DBT (28 % n) |

Die Estergemische aus Tabelle 2 wurden auf anwendungsrelevante Eigenschaften hin untersucht.

Beispiel 9: Tieftemperaturviskosität der Estergemische

Die Viskositäten der Estergemische wurden an einem Rheometer Physica MCR 302 (Firma Anton Paar Germany GmbH) bestimmt. Das Rheometer hat neben der Standardausrüstung folgende Zusatzeinrichtungen:

| | |
|---|---|
| Temperiereinrichtung: | CTD 450 Fa. Anton Paar Germany GmbH |
| Messsystem: | PP 25 Platte Platte System Fa. Anton Paar Germany GmbH |
| Stickstoffverdampfer: | EVU 10 Fa. Anton Paar Germany GmbH |
| Gegentemperierthermostat: | Viscotherm VT2 Fa. Anton Paar Germany GmbH |
| Stickstoffbehälter: | Apollo 100 Cryotherm GmbH & Co KG |

Die Messungen wurden bei 25 °C gestartet. Das Platte-Platte Messsystem wurde, nachdem der Nullpunkt gesetzt wurde, auf eine Messspaltbreite von 0,5 mm eingestellt. Mit Hilfe einer Einwegpipette wurden die Proben auf das Plattesystem gegeben. In der Trimmposition wurde überprüft, ob der Messspalt mit ausreichend Probe gefüllt war. Die Temperiereinrichtung CTD 450 wurde geschlossen und mit Hilfe des Thermostaten wurde die Ummantelung der Temperiereinrichtung auf 23 °C eingestellt.

Ein Messprogramm mit folgenden Parametern wurde in der Software erstellt:

**Abschnitt 1**

| | |
|---|---|
| Zeitvorgabe | 78 Messpunkte, Abschnittsdauer 13 min |
| Messprofil | |
| - Deformation | Amplitude gamma 0,1%; Frequenz f = 10 Hz |
| - Normalkraft | $F_N$ = 0 N |
| - Temperatur | $T_{[-1]}$ = +25....-40 °C linear |

**Abschnitt 2**

| | |
|---|---|
| Zeitvorgabe | 3 Messpunkte, Abschnittsdauer 3 min |
| Messprofil | |
| - Normalkraft | $F_N$ = 0 N |
| - Temperatur | $T_{[-1]}$ = -40 °C |

**Abschnitt 3**

| | |
|---|---|
| Zeitvorgabe | 78 Messpunkte, Abschnittsdauer 13 min |
| Messprofil | |
| - Deformation | Amplitude gamma 0,1%; Frequenz f = 10 Hz |
| - Normalkraft | $F_N$ = 0 N |
| - Temperatur | $T_{[-1]}$ = -40....+25 °C linear |

Mit Hilfe der Flüssigstickstoff-Temperierung wurde die CTD 450 nun auf 25 °C temperiert. Die Messung startete nach einer 1-minütigen Temperaturkonstanz bei 25 °C ± 1 °C.

Die Auswertung der Viskositäten erfolgte über die Rheologie Software Rheoplus 3.6.1. Zuerst wurde die Kurve im Auswerteprogramm über die automatisierten Auswertemodule "Smoothing" und "Merging" von Messfehlern befreit. Durch einen weiteren Auswerteschritt "Interpolation" wurde die Temperatur bei 1000 Pa·s wie folgt bestimmt: Logarithmische Interpolation der Temperatur (x-Achse) im Verhältnis zu dem "Betrag Viskosität" (y-Achse) der geglätteten Kurve. In Tabelle 3 sind die auf diese Weise bestimmten Temperaturen, bei denen die Estergemische eine Viskosität von 1000 Pa·s aufweisen, zusammengestellt.

Das Butylestergemisch der Terephthalsäure mit 28 % linearen Butylresten (DBT 28 % n, Beispiel 8) weist einen Schmelzpunkt von über 25 °C auf. Auf eine Bestimmung der Tieftemperaturviskosität wurde deshalb verzichtet.

Die im Messabschnitt 3 erhaltenen Viskositätswerte wurden gegen die Temperatur aufgetragen (Abbildung 1). Der Abbildung 1 lassen sich folgende Informationen entnehmen:

- Die untersuchten Pentylestergemische mit 38 %, 53 % und 58 % linearen Pentylresten (DPT 38 % n, DPT 53 % n, DPT 58 % n) zeigen keine nennenswerte Veränderung der Viskosität im untersuchten Temperaturintervall (-40 °C (+ Übersteuerung der Kühlung) bis 25 °C). Die Viskositäten liegen durchgehend bei Werten unterhalb von 100 Pa·s. Die Estergemische sind somit im gesamten Temperaturbereich problemlos pumpbar.
- Das Pentylestergemisch mit 21 % linearen Pentylresten (DPT 21 % n) verfestigt sich (vermutlich aufgrund kinetischer Faktoren) erst beim Erwärmen (bei -32 °C) um sich dann bei ca. 5 °C wieder zu verflüssigen (Viskosität unter 1000 Pa·s).
- Das Pentylestergemisch mit 77 % linearen Pentylresten (DPT 77 % n) verflüssigt sich (Viskosität kleiner 1000 Pa·s) bei ca. 3 °C.
- Die Butylestergemische mit 54 % bzw. 78 % linearen Butylresten (DBT 54 % n bzw. DBT 78 % n) verflüssigen sich (Viskosität keiner 1000 Pa·s) bei ca. -5 °C bzw. bei ca. 0 °C.
- Erklärung der Schultern: Schwankungen aufgrund von Dichteänderungen der Proben bei variablem Messspalt (Normalkraft auf Messsystem $F_N = 0$).

Tabelle 3: Temperatur bei Erreichen einer Viskosität von 1000 Pa·s aus Interpolation

| Estergemisch (Anteil linearer Alkylreste in %) | Temperatur bei 1000 Pa·s [°C] |
|---|---|
| Dipentylterephthalat (21 % n) | 5,3 |
| Dipentylterephthalat (38 % n) | < 40,0 |
| Dipentylterephthalat (53 % n) | < 40,0 |
| Dipentylterephthalat (58 % n) | < 40,0 |
| Dipentylterephthalat (77 % n) | 3,2 |
| | |
| Dibutylterephthalat (54 % n) | -5,1 |
| Dibutylterephthalat (78 % n) | 0,1 |

<u>Beispiel 10: DSC unterschiedlicher Dipentylterephthalatgemische</u>

Die DSC-Messungen (Dynamische Differenzkalorimetrie, englisch: Differential Scanning Calorimetry) basieren auf dem bewährten Boersma- oder Wärmestromprinzip, in welchem der Wärmefluss einer Probe und einer Referenz verglichen werden. Ein hoch sensitiver Keramik-Sensor wird für die Messung der Differenz zwischen den Wärmeströmen benutzt. Mit diesem Prinzip können sehr geringe thermische Änderungen in der Probe ermittelt werden. Beispielsweise können so Glasübergänge, Schmelzübergänge, Kristallisationsübergänge, Siedeübergänge oder Zersetzungsübergänge gemessen werden. Nicht selten kommt es vor, dass thermische Übergänge dicht beieinander liegen. Durch Veränderungen einiger Messparameter können diese aufgeschlüsselt werden.

Die Proben wurden in einem Aluminium-Tiegel unter Stickstoff mit einer DSC 1 der Firma Mettler Toledo unter folgenden Einstellungen vermessen:

| | |
|---|---|
| Drygas Stickstoff (Trockengas): | ca. 45 $l_s$/min |
| Gas Stickstoff (Spülgas): | ca. 180 $l_s$/min |
| Kühlung: | flüssiger Stickstoff (Zusatzbehälter 1,5 bar) |
| Methode: Rekristallisation | [1] 25,0 bis 80,0 °C, -1,0 K/min |
| | [2] -80,0 bis -120,0 °C, -25,0 K/min |
| | [3] -120,0 °C, 3,00 min |
| | [4] -120,0 bis 100,0 °C, 10,0 K/min |
| | [5] 100 bis 25,0 °C, -30,0 K/min |

Die synchronisierten Aufheizkurven finden sich als Abbildungen 2 bis 8. Der Wärmestrom in Watt pro Gramm Probe ist in Abhängigkeit der Temperatur in Grad Celsius aufgetragen (↑endo).. Die Auswertung der Kurven ist in Tabelle 4 Erwartungswerten gegenübergestellt.

Tabelle 4: Auswertung DSC der Dipentylterephthalatgemische

| Estergemisch (Anteil linearer Alkylreste) | Schmelzpunkt (onset) | Schmelzenthalpie [J/g] gemessen ($H_{gem}$) | Schmelzenthalpie [J/g] erwartet ($H_{erw}$) gemäß 1 | Boeck-Faktor (BF) gemäß 2 |
|---|---|---|---|---|
| Dipentylterephthalat (0 % n) | 15,1 °C | 75,8 | 75,8 | 100 |
| Dipentylterephthalat (21 % n) | -13,6 °C | 86,1 | 93,4 | 92 |
| Dipentylterephthalat (38 % n) | -13,7 °C | 1,3 | 107,7 | 1 |
| Dipentylterephthalat (53 % n) | - | - | 120,3 | 0 |
| Dipentylterephthalat (58 % n) | -15,1 °C | 3,7 | 124,5 | 3 |
| Dipentylterephthalat (77 % n) | 3,6 °C | 99,1 | 140,5 | 71 |
| Dipentylterephthalat (100 % n) | 21,1 °C | 169,1 | 169,1 | 100 |

1: Berechnung der erwarteten Schmelzenthalpie $H_{erw}$:

$$H_{erw} = H_{D^nPT} \cdot x^n_{Pentyl} + H_{D(2-Methylbutyl)PT} \cdot x_{2-Methylbutyl}$$

mit

$H_{D^nPT}$: Schmelzenthalpie von Di-*n*-pentylterephthalat
$x^n_{Pentyl}$: Molenbruch der *n*-Pentylreste an allen Pentylresten im jeweiligen DPT
$H_{D(2\text{-}Methylbutyl)PT}$: Schmelzenthalpie von Di(2-methylbutyl)terephthalat
$X_{2\text{-}Methylbutyl}$: Molenbruch der 2-Methylbutylreste an allen Pentylresten im jeweiligen DPT

2: Berechnung des Boeck-Faktors BF:

$$BF = \frac{H_{gem}}{H_{erw}} \cdot 100$$

mit

$H_{erw}$: berechnet wie unter 1 beschrieben
$H_{gem}$ gemessene Enthalpie (bei der in der Tabelle angegebenen Temperatur)

Beispiel 11: Erstarrungs-und Trübungsverhalten der Estergemische

Die Bestimmung des Erstarrungsverhaltens von flüssigen Substanzen erfolgte in Anlehnung an ISO 1392 und entspricht weitestgehend der Gefriertemperaturmethode nach OECD (guideline 102, Abschnitt 19) beziehungsweise der EU Test-Methode A.1 (Abschnitt 1.4.3). Das Erstarrungsverhalten wird durch Abkühlung der flüssigen Probe unter Rühren und Aufzeichnen der Temperatur ermittelt. Beim Erstarren der Probe oder von Probenanteilen bleibt die Temperatur kurzzeitig konstant. Dieses "Temperaturplateau" wird dokumentiert. Zur Bestimmung des Erstarrungsverhaltens wurden 10 ml Probenflüssigkeit in das Probengefäß vorgelegt, das Thermoelement in die Flüssigkeit getaucht und mittels eines Magnetrührers gerührt. Der Thermostat wurde auf -50 °C gestellt und die Probe abgekühlt. Zur Kühlung wird ein Julabo FN32 mit dem Kühlmittel Ethanol, betrieben im Standardprogramm, eingesetzt. Während des Abkühlens wurde die Temperatur aufgezeichnet. Es wurden 2 gültige Bestimmungen durchgeführt und der Mittelwert in Tabelle 5

eingetragen.

Der Cloudpoint wurde in Anlehnung an die DIN EN 23015 bestimmt. Diese Norm gilt im strengen Sinne nur für Mineralölerzeugnisse und legt ein Verfahren zur Bestimmung des Cloudpoints von Mineralölerzeugnisse fest. Der Cloudpoint ist "die Temperatur, bei der eine Wolke von Paraffinkristallen (Trübung) erstmalig in einer Flüssigkeit auftritt, wenn diese unter den festgelegten Prüfbedingungen abgekühlt wird" (DIN EN 23015:1994). Der in Tabelle 5 angegebene Cloudpoint entspricht der Temperatur, bei welcher zum ersten Mal eine Trübung im Probegefäß beobachtet wurde.

Tabelle 5: Erstarrungsverhalten und Cloudpunkt der Estergemische

| Estergemische (Anteil linearer Alkylreste) | Cloudpunkt [°C] | Temperaturniveau Erstarrungsverhalten [°C] |
|---|---|---|
| Dipentylterephthalat (21 % n) | -5,4 °C | -9,6 °C |
| Dipentylterephthalat (38 % n) | nein | -26,9 °C |
| Dipentylterephthalat (53 % n) | -27,9 °C | n.b. |
| Dipentylterephthalat (58 % n) | nein | -28,2 °C |
| Dipentylterephthalat (77 % n) | -1,6 °C | -1,1 °C |
| | | |
| Dibutylterephthalat (28 % n) | 35,5 °C | 33,8 °C |
| Dibutylterephthalat (54 % n) | -5,6 °C | -9,7 °C |
| Dibutylterephthalat (78 % n) | -10,5 °C | -0,1 °C |
| n.b.: nicht bestimmt<br>nein: bei der Messung wurde kein Cloudpunkt detektiert | | |

Beispiel 12: Plastisolherstellung

Es wurden PVC-Plastisole hergestellt, wie sie beispielsweise zur Fertigung von Deckstrichfilmen für Fußbodenbeläge verwendet werden. Die Rezepturen der Plastisole sind in Tabelle 6 aufgelistet.

Tabelle 6: Plastisolrezeptur

| | |
|---|---|
| PVC (Vestolit B 7021 - Ultra; Fa. Vestolit) | 100 |
| jeweiliger Weichmacher aus Tabelle 2 | 50 |
| epoxidiertes Sojabohnenöl als Co-Stabilisator (Drapex 39, Fa. Galata) | 3 |
| Thermostabilisator auf Ca/Zn-Basis (Reagens CLX/759/6PF, Fa. Reagens) | 2 |
| Angaben in phr (phr = parts per hundred parts resin) | |

Zuerst wurden die flüssigen und dann die pulverförmigen Bestandteile in einen PE-Becher eingewogen. Vor der Zugabe in den PE-Becher war das Dibutylterephthalat (DBT 28 % n) aufgeschmolzen worden, während die anderen Weichmacher vor der Zugabe auf 25 °C temperiert worden waren. Von Hand wurde die Mischung mit einem Salbenspatel so eingerührt, dass kein unbenetztes Pulver mehr vorhanden war. Der Mischbecher wurde dann in die Klemmvorrichtung eines Vakuierungsgefäßes von einem Dissolverrührer (Firma Kreiss) eingespannt. Nach dem Eintauchen des Rührers in die Mischung wurde die Evakuierungseinheit geschlossen und mit Hilfe einer Vakuumpumpe ein Unterdruck von unter 20 mbar erzeugt. Die Mischung wurde gerührt, wobei die Drehzahl wurde von ca. 400 auf 2000 Umdrehungen pro Minute erhöht wurde. Es wurde so lange bei hoher Drehzahl gerührt, bis die Temperatur an der Digitalanzeige des Thermofühlers 30 °C erreichte. Damit war sichergestellt, dass die Homogenisierung des Plastisols bei einem definierten Energieeintrag erreicht wurde. Danach wurde die Drehzahl wieder auf 400 Umdrehungen pro Minute abgesenkt und das Plastisol für weitere 9 Minuten entlüftet. Nach der Entlüftung wurde zuerst der Rührer abgestellt und das Evakuierungsgefäß wurde wieder an den Raumdruck angeglichen. Das fertige Plastisol wurde sofort für weitere Untersuchungen in einem Klimaschrank auf 25 °C temperiert.

Beispiel 13: Geliertemperatur der Plastisole

Die Untersuchung des Gelierverhaltens der Plastisole wurde mit einem Rheometer Physica MCR 101 (Firma

Anton Paar Germany GmbH) im Oszillationsmodus mit einem Platte-Platte Messsystem (PP25) vorgenommen. Eine zusätzliche Temperierhaube wurde an das Gerät angeschlossen, um eine homogene Wärmeverteilung und eine gleichmäßige Probentemperatur zu erreichen.

Folgende Parameter wurden eingestellt:

| | | |
|---|---|---|
| Modus: | Temperatur-Gradient | |
| | Start-Temperatur: | 25 °C |
| | End-Temperatur: | 180 °C |
| | Heiz/Kühlrate: | 5 °C / min |
| | Oszillations-Frequenz: | 4 - 0,1 Hz Rampe logarithmisch |
| | Kreisfrequenz Omega: | 10 $s^{-1}$ |
| | Anzahl Messpunkte: | 63 |
| | Messpunktdauer: | 0,5 min |
| | Automatische Spaltnachführung F : | 0 N |
| | Konstante Messpunktdauer | |
| | Spaltweite | 0,5 mm |

Durchführung der Messung:

Auf die untere Messsystemplatte wurden mit dem Spatel einige Gramm des zu messenden Plastisols luftblasenfrei aufgetragen. Dabei wurde darauf geachtet, dass nach dem Zusammenfahren des Messsystems etwas Plastisol gleichmäßig aus dem Messsystem herausquellen konnte (nicht mehr als 6 mm rundum). Anschließend wurde die Temperierhaube über der Probe positioniert und die Messung gestartet. Bestimmt wurde die sogenannte komplexe Viskosität des Plastisols nach 24 h (nach Lagerung des Plastisols bei 25 °C in einem Temperierschrank der Firma Memmert) in Abhängigkeit von der Temperatur.

Als Maß für die Gelierung wurde ein deutlicher Anstieg der komplexen Viskosität betrachtet. Als Vergleichswert wurde daher die Temperatur bei Erreichen einer Plastisolviskosität von 1000 Pa·s verwendet.

Tabelle 7: Gelierung der Plastisole nach 24 h, Temperatur in °C bei Erreichen einer Plastisolviskosität von 1000 Pa·s (kurz: Geliertemperatur)

| Estergemisch (Anteil linearer Alkylreste in %) | Geliertemperatur [°C] |
|---|---|
| Dipentylterephthalat (21 % n) | 71,7 |
| Dipentylterephthalat (38 % n) | 70,6 |
| Dipentylterephthalat (53 % n) | 70,2 |
| Dipentylterephthalat (58 % n) | 70,3 |
| Dipentylterephthalat (77 % n) | 70,0 |
| | |
| Dibutylterephthalat (28 % n) | 67,5 |
| Dibutylterephthalat (54 % n) | 65,7 |
| Dibutylterephthalat (78 % n) | 65,0 |

Die Plastisole der Pentylester weisen eine von der Isomerenverteilung der Pentylreste unabhängige, wenig variierende Geliertemperatur auf, während die Geliertemperatur der Plastisole der Butylester über einen deutlich größeren Bereich streut.

Beispiel 14: Messung der Plastisolviskosität

Die Messung der Viskositäten der in Beispiel 12 hergestellten Plastisole wurden mit einem Rheometer Physica MCR 301 (Firma Anton Paar Germany GmbH) mit Hilfe der zugehörigen "Rheoplus Software" wie folgt durchgeführt.

Das Plastisol wurde im Vorratsbehälter nochmals mit einem Spatel umgerührt und in dem Messsystem Z3 (DIN 25 mm) gemäß Bedienungsanleitung vermessen. Die Messung verlief bei 25 °C automatisch über die oben genannte

Software. Folgende Punkte wurden angesteuert:

Eine Vorscherung von 100 $s^{-1}$ für den Zeitraum von 60 s, bei der keine Messwerte aufgenommen wurden.

Eine Abwärtsrampe, beginnend bei 200 $s^{-1}$ bis herunter zu 0,1 $s^{-1}$, aufgeteilt in eine logarithmische Reihe mit 30 Schritten mit jeweils 5 s Messpunktdauer.

Die Aufbereitung der Messdaten wurde nach der Messung automatisch von der Software durchgeführt. Dargestellt wurde die Viskosität in Abhängigkeit von der Schergeschwindigkeit. Die Messungen wurden jeweils nach 2 h, 24 h und 7 Tagen durchgeführt. Zwischen diesen Zeitpunkten wurde die Paste bei 25 °C gelagert.

Die Ergebnisse der Messung sind in der Abbildungen 9 und 10 dargestellt. Die Abbildung 9 veranschaulicht die Plastisolviskosität der einzelnen Pasten nach 7-tägiger Lagerung bei 25 °C in Abhängigkeit der Schergeschwindigkeit. Die Abbildung 10 stellt die Veränderung der Plastisolviskosität nach jeweils 1- tägiger und 7-tägiger Lagerung (bei 25 °C) basierend auf der Viskosität des nach ihrer Herstellung lediglich 2 Stunden auf 25 °C temperierten Plastisols bei Schergeschwindigkeiten von 1 $s^{-1}$, 10 $s^{-1}$ und 100 $s^{-1}$ in % dar (Darstellung in %) und erlaubt damit eine Aussage über das Eindickverhalten der Plastisole.

Die Plastisole der Pentylestergemische weisen nach Lagerung im Vergleich zu den Plastisolen der Butylestergemische niedrigere Viskositäten auf. Zudem zeichnen sich die Viskositäten der Pentylester-Plastisole durch eine hohe Konstanz - unabhängig von der Isomerenverteilung der Pentylreste - aus (Abbildung 9). Innerhalb von 7 Tagen dicken die Pentylester-Plastisole um weniger als 130 % ein, während die Butylester-Plastisole ohne Ausnahme eine wesentliche höhere Eindicktendenz aufweisen (Abbildung 10). Somit zeichnen sich die Pentylester-Plastisole im Vergleich zu den Butylester-Plastisolen sowohl in Abhängigkeit der Zeit als auch in Abhängigkeit der Isomerenverteilung der Alkylreste durch konstantere und damit verlässlichere Eigenschaften auf.

## Patentansprüche

**1.** Gemisch umfassend

- Di*i*sopentylterephthalat, dessen Pentylreste zu mehr als 25 Mol-% und gleichzeitig zu weniger als 60 Mol-% *n*-Pentylreste sind, wobei mindestens 60 Mol-% der im Di*i*sopentylterephthalat gebundenen verzweigten isomeren Pentylreste 2-Methylbutylreste sind,
- mindestens einen zusätzlichen Weichmacher ausgewählt aus der Gruppe der Alkylbenzoate, Dialkyladipate, Glycerinester, Citronensäuretrialkylester, acylierten Citronensäuretrialkylester, Trialkyltrimellitate, Glykoldibenzoate, Ester der Furandicarbonsäure, Dialkanoylester von Dianhydrohexitolen und Dialkylester der 1,2-, 1,3- oder 1,4-Cyclohexandicarbonsäure.

**2.** Gemisch gemäß Anspruch 1, , **dadurch gekennzeichnet, dass** das Di*i*sopentylterephthalat bei Temperaturen oberhalb von 10 °C eine Viskosität kleiner 1000 Pa·s aufweist.

**3.** Produkt, insbesondere ein Unterbodenschutz, eine Tapete, ein Kunstleder, eine Gewebebeschichtung oder ein Fußboden umfassend Di*i*sopentylterephthalat, dessen Pentylreste zu mehr als 25 Mol-% und gleichzeitig zu weniger als 60 Mol-% *n*-Pentylreste sind, wobei mindestens 60 Mol-% der im Di*i*sopentylterephthalat gebundenen verzweigten isomeren Pentylreste 2-Methylbutylreste sind, oder ein Gemisch gemäß Anspruch 1 oder 2.

**4.** Verwendung eines Gemisches gemäß einem der Ansprüche 1 oder 2 als Weichmacher in Kunststoffzusammensetzungen, insbesondere in PVC-haltigen Kunststoffzusammensetzungen.

**5.** Verwendung von Di*i*sopentylterephthalat, dessen Pentylreste zu mehr als 25 Mol-% und gleichzeitig zu weniger als 60 Mol-% *n*-Pentylreste sind, wobei mindestens 60 Mol-% der im Di*i*sopentylterephthalat gebundenen verzweigten isomeren Pentylreste 2-Methylbutylreste sind, oder von einem Gemisch gemäß einem der Ansprüche 1 oder 2 zur Viskositätserniedrigung und/oder zur Verbesserung der Lagerfähigkeit von Plastisolen.

**6.** Verwendung von Di*i*sopentylterephthalat, dessen Pentylreste zu mehr als 25 Mol-% und gleichzeitig zu weniger als 60 Mol-% *n*-Pentylreste sind, wobei mindestens 60 Mol-% der im Di*i*sopentylterephthalat gebundenen verzweigten isomeren Pentylreste 2-Methylbutylreste sind, oder von einem Gemisch gemäß einem der Ansprüche 1 oder 2 bei der Herstellung von Plastisolen bei Umgebungstemperaturen, welche nicht verlässlich oberhalb von 20 °C liegen.

## Claims

1. Mixture comprising

   - diisopentyl terephthalate, the pentyl radicals of which are n-pentyl radicals to an extent of more than 25 mol% and at the same time less than 60 mol%, wherein at least 60 mol% of the branched isomeric pentyl radicals incorporated in the diisopentyl terephthalate are 2-methylbutyl radicals,
   - at least one additional plasticizer selected from the group of alkyl benzoates, dialkyl adipates, glycerine esters, trialkyl citrates, acylated trialkyl citrates, trialkyl trimellitates, glycol dibenzoates, esters of furandicarboxylic acid, dialkanoyl esters of dianhydrohexitols and dialkyl esters of 1,2-, 1,3- or 1,4-cyclohexanedicarboxylic acid.

2. Mixture according to Claim 1, **characterized in that** the diisopentyl terephthalate has a viscosity of less than 1000 Pa·s above 10°C.

3. Product, particularly an underbody protection, a wallcovering, a synthetic leather, a fabric coating or a flooring comprising diisopentyl terephthalate, the pentyl radicals of which are n-pentyl radicals to an extent of more than 25 mol% and at the same time less than 60 mol%, wherein at least 60 mol% of the branched isomeric pentyl radicals incorporated in the diisopentyl terephthalate are 2-methylbutyl radicals, or a mixture according to Claim 1 or 2.

4. Use of a mixture according to either of Claims 1 or 2 as plasticizer in plastic compositions, particularly in PVC-containing plastic compositions.

5. Use of diisopentyl terephthalate, the pentyl radicals of which are n-pentyl radicals to an extent of more than 25 mol% and at the same time less than 60 mol%, wherein at least 60 mol% of the branched isomeric pentyl radicals incorporated in the diisopentyl terephthalate are 2-methylbutyl radicals, or of a mixture according to either of Claims 1 or 2 for lowering viscosity and/or improving storability of plastisols.

6. Use of diisopentyl terephthalate, the pentyl radicals of which are n-pentyl radicals to an extent of more than 25 mol% and at the same time less than 60 mol%, wherein at least 60 mol% of the branched isomeric pentyl radicals incorporated in the diisopentyl terephthalate are 2-methylbutyl radicals, or of a mixture according to either of Claims 1 or 2 in the production of plastisols at ambient temperatures which are not reliably above 20°C.

## Revendications

1. Mélange comprenant

   - du téréphtalate de di*i*sopentyle, dont les radicaux pentyle sont à raison de plus de 25 % en moles et en même temps de moins de 60 % en moles des radicaux *n*-pentyle, au moins 60 % en moles des radicaux pentyle isomères ramifiés, liés dans le téréphtalate de di*i*sopentyle, étant des radicaux 2-méthylbutyle,
   - au moins un plastifiant supplémentaire choisi dans le groupe des benzoates d'alkyle, adipates de dialkyle, esters de glycérol, citrates de trialkyle, citrates de trialkyle acylés, trimellitates de trialkyle, dibenzoates de glycol, esters de l'acide furanedicarboxylique, esters de dialcanoyle de dianhydrohexitols et esters dialkyliques de l'acide 1,2-, 1,3- ou 1,4-cyclo-hexanedicarboxylique.

2. Mélange selon la revendication 1, **caractérisé en ce que** le téréphtalate de diisopentyle présente à des températures supérieures à 10 °C une viscosité inférieure à 1 000 Pa.s.

3. Produit, en particulier une protection de bas de caisse, une tapisserie, un cuir artificiel, un revêtement textile ou un plancher comprenant du téréphtalate de diisopentyle dont les radicaux pentyle sont à raison de plus de 25 % en moles et en même temps de moins de 60 % en moles des radicaux *n*-pentyle, au moins 60 % en moles des radicaux pentyle isomères ramifiés, liés dans le téréphtalate de di*i*sopentyle, étant des radicaux 2-méthylbutyle, ou un mélange selon la revendication 1 ou 2.

4. Utilisation d'un mélange selon l'une quelconque des revendications 1 et 2 en tant que plastifiant dans des compositions de matière plastique, en particulier des compositions de matière plastique contenant du PVC.

5. Utilisation de téréphtalate de di*i*sopentyle dont les radicaux pentyle sont à raison de plus de 25 % en moles et en

même temps de moins de 60 % en moles des radicaux *n*-pentyle, au moins 60 % en moles des radicaux pentyle isomères ramifiés, liés dans le téréphtalate de di*i*sopentyle, étant des radicaux 2-méthylbutyle, ou d'un mélange selon l'une quelconque des revendications 1 et 2, pour l'abaissement de la viscosité et/ou pour l'amélioration de l'aptitude au stockage de plastisols.

**6.** Utilisation de téréphtalate de di*i*sopentyle dont les radicaux pentyle sont à raison de plus de 25 % en moles et en même temps de moins de 60 % en moles des radicaux *n*-pentyle, au moins 60 % en moles des radicaux pentyle isomères ramifiés, liés dans le téréphtalate de di*i*sopentyle, étant des radicaux 2-méthylbutyle, ou d'un mélange selon l'une quelconque des revendications 1 et 2, dans la production de plastisols à des températures ambiantes qui ne sont pas de façon sûre supérieures à 20 °C.

Abbildung 1: Tieftemperaturviskosität der Estergemische

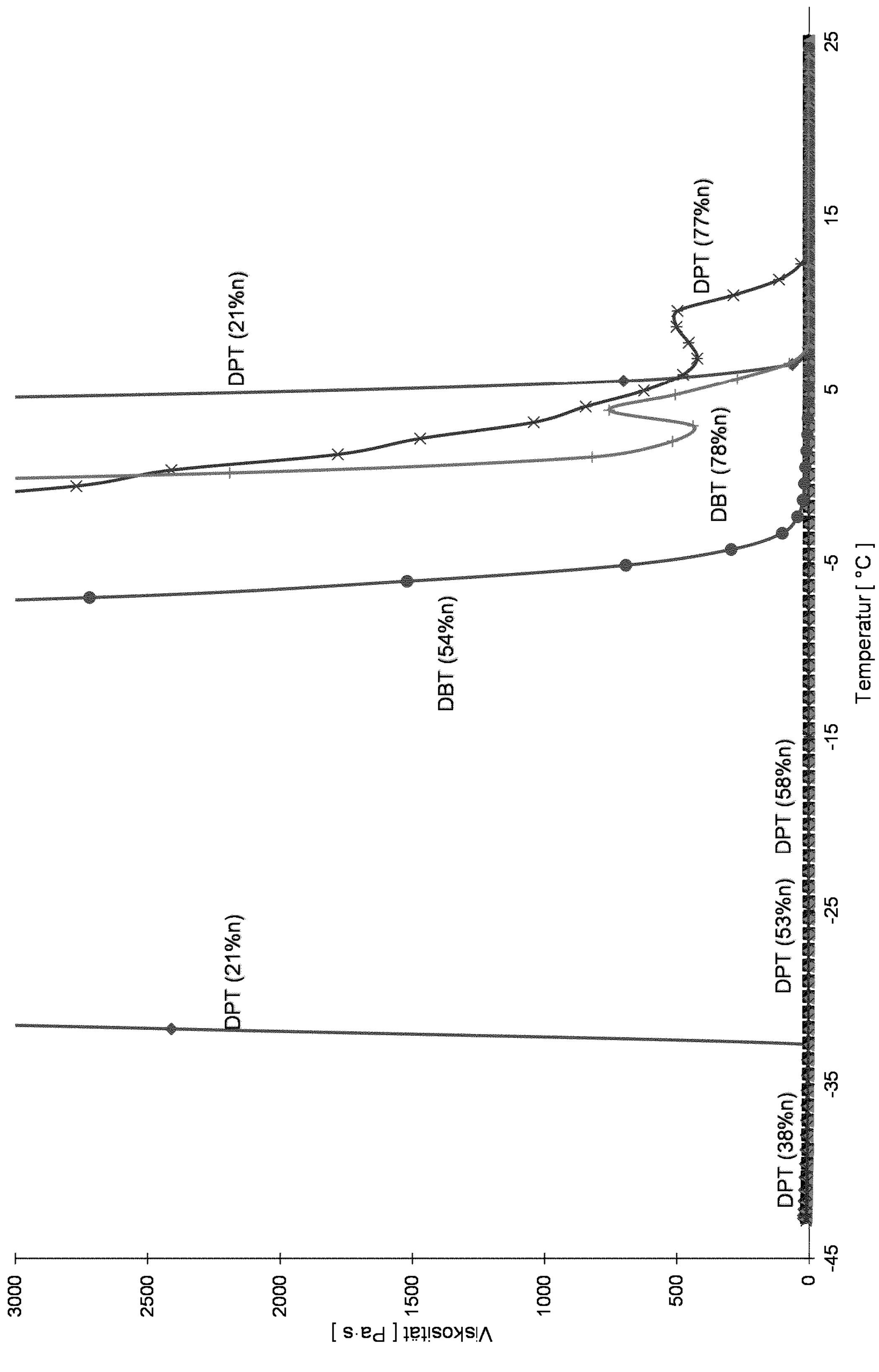

Abbildung 2: DSC von DPT (0%n, ↑endo)

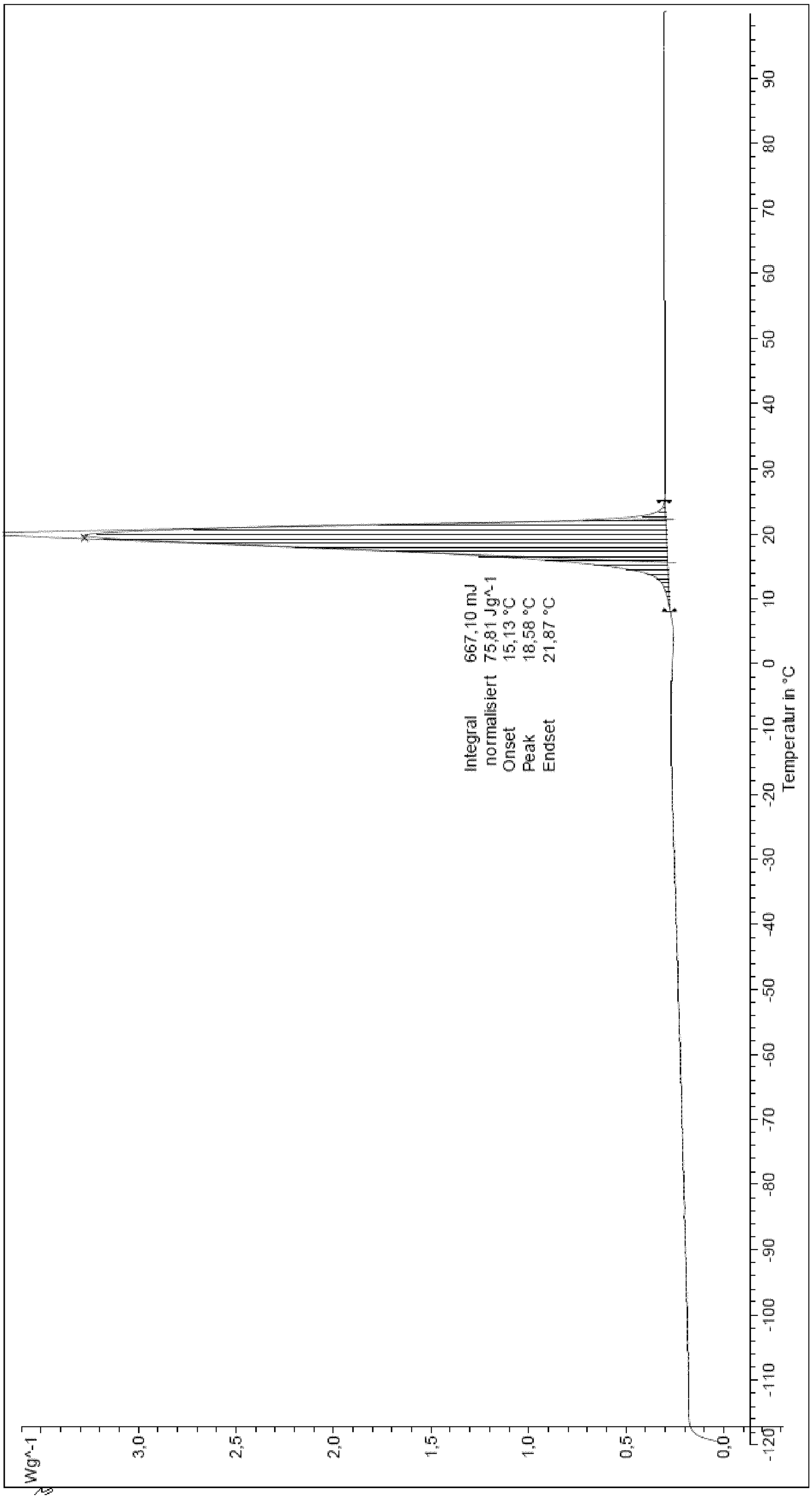

Abbildung 3: DSC von DPT (21%n, ↑endo)

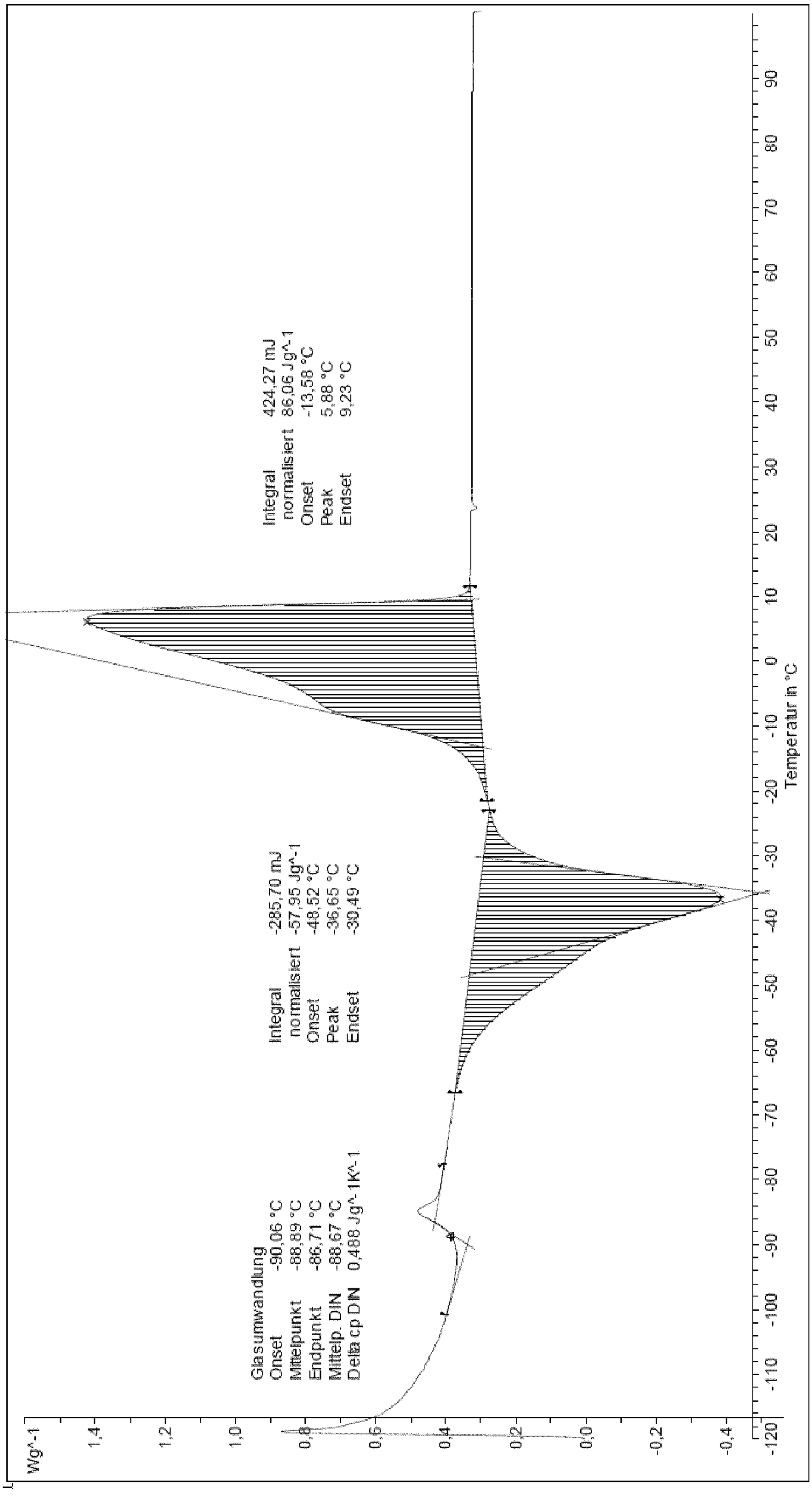

Abbildung 4: DSC von DPT (38%n, ↑endo)

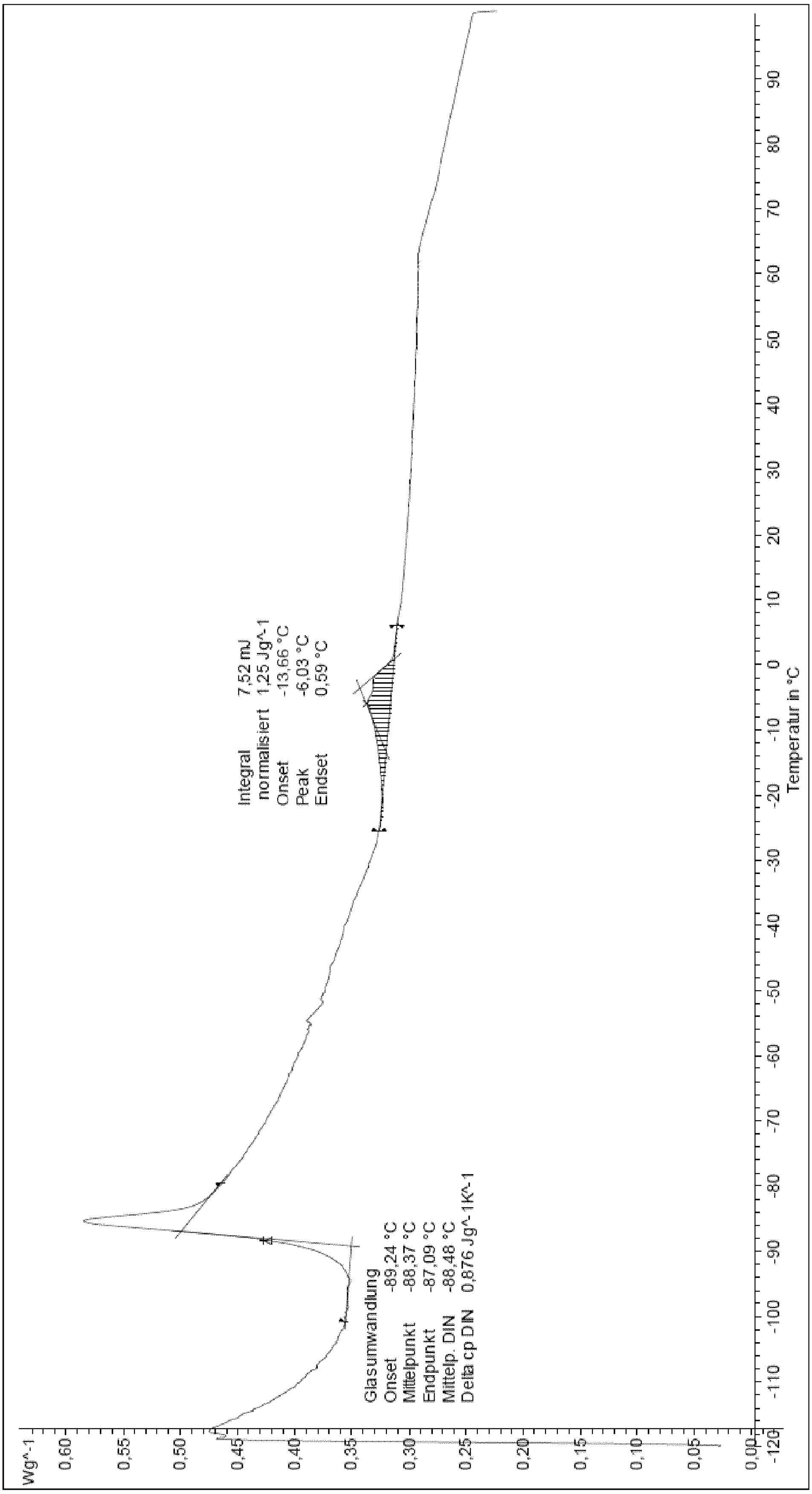

Abbildung 5: DSC von DPT (53%n, ↑endo)

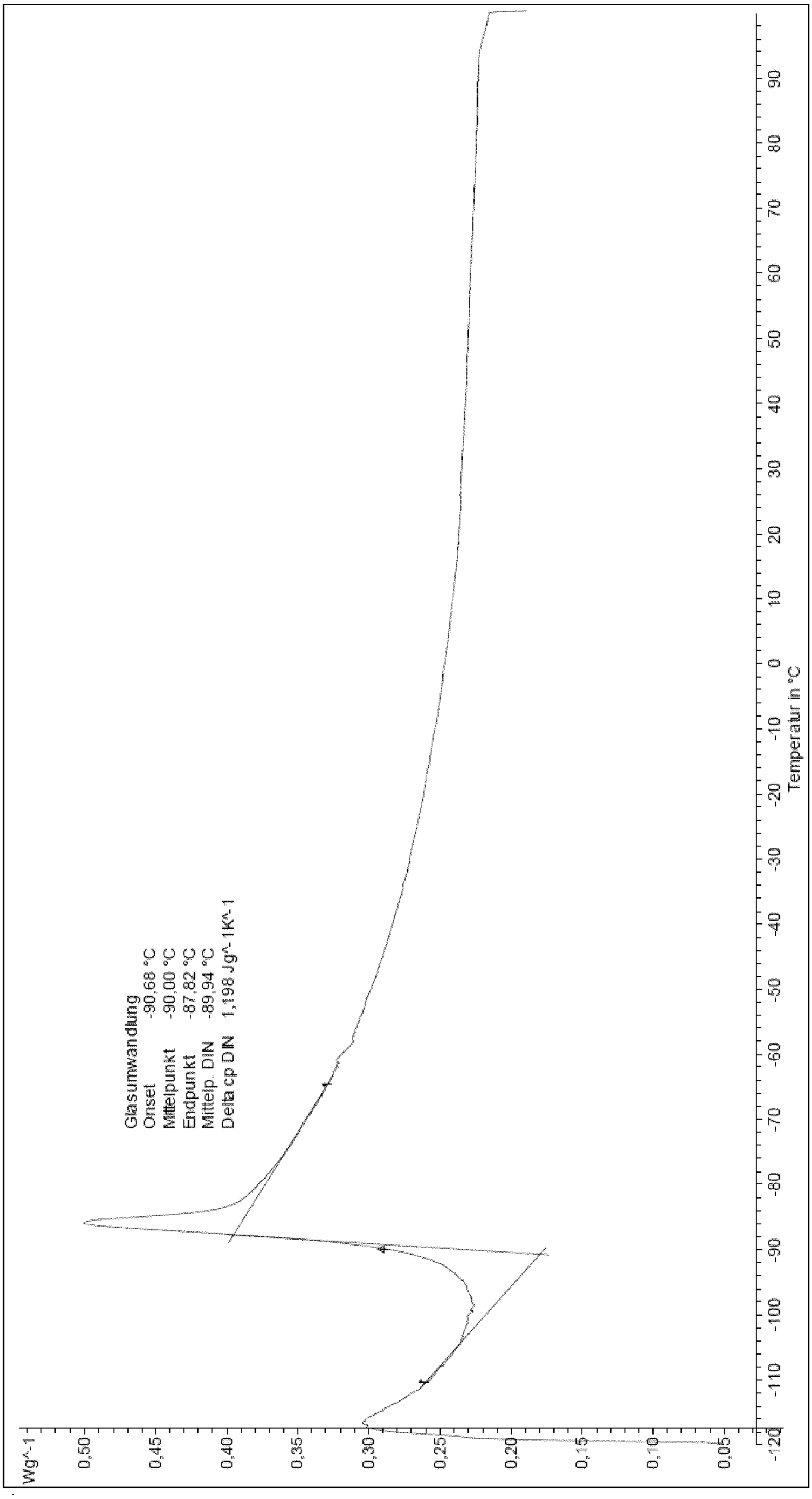

Abbildung 6: DSC von DPT (58%n, ↑endo)

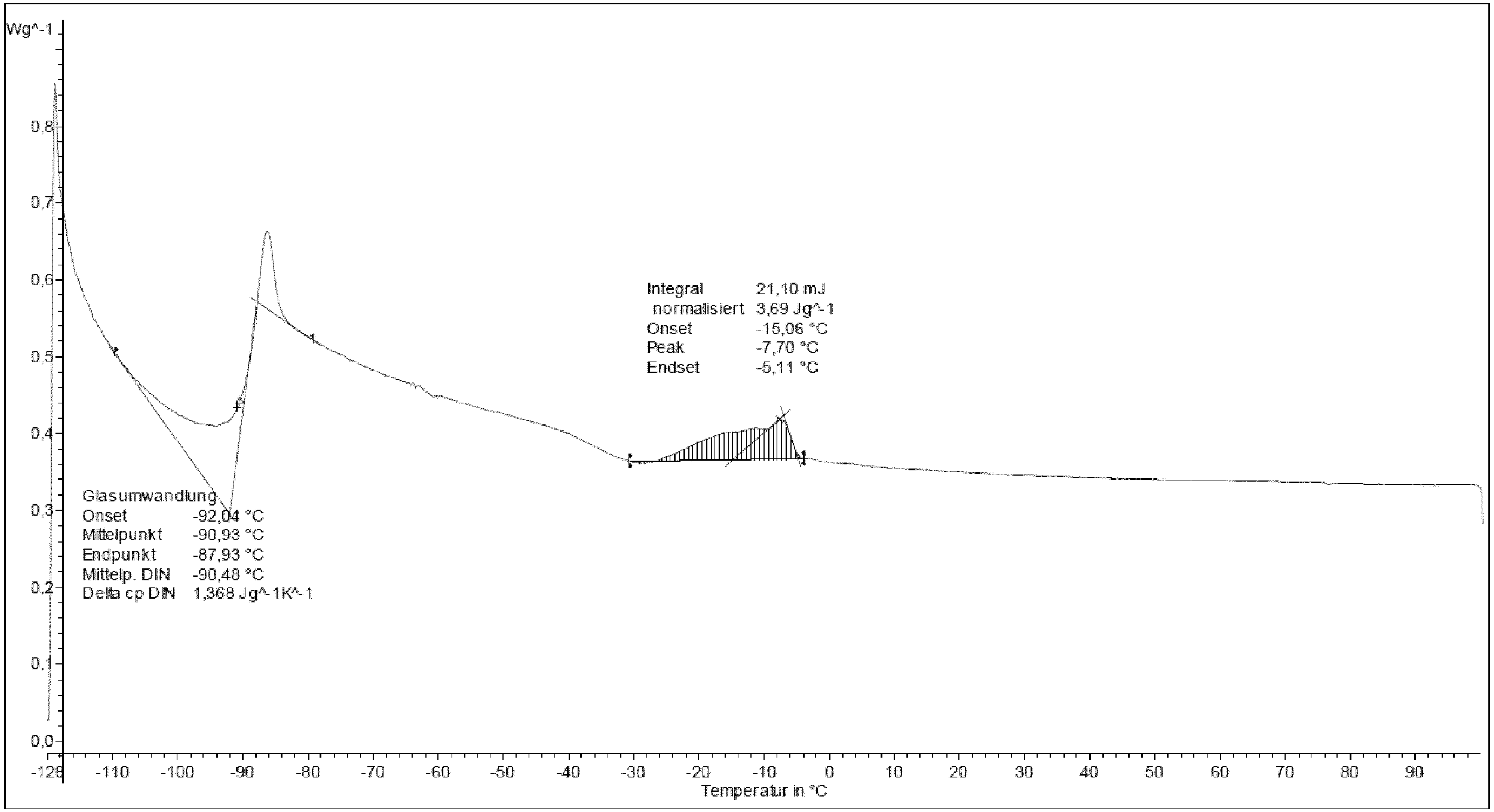

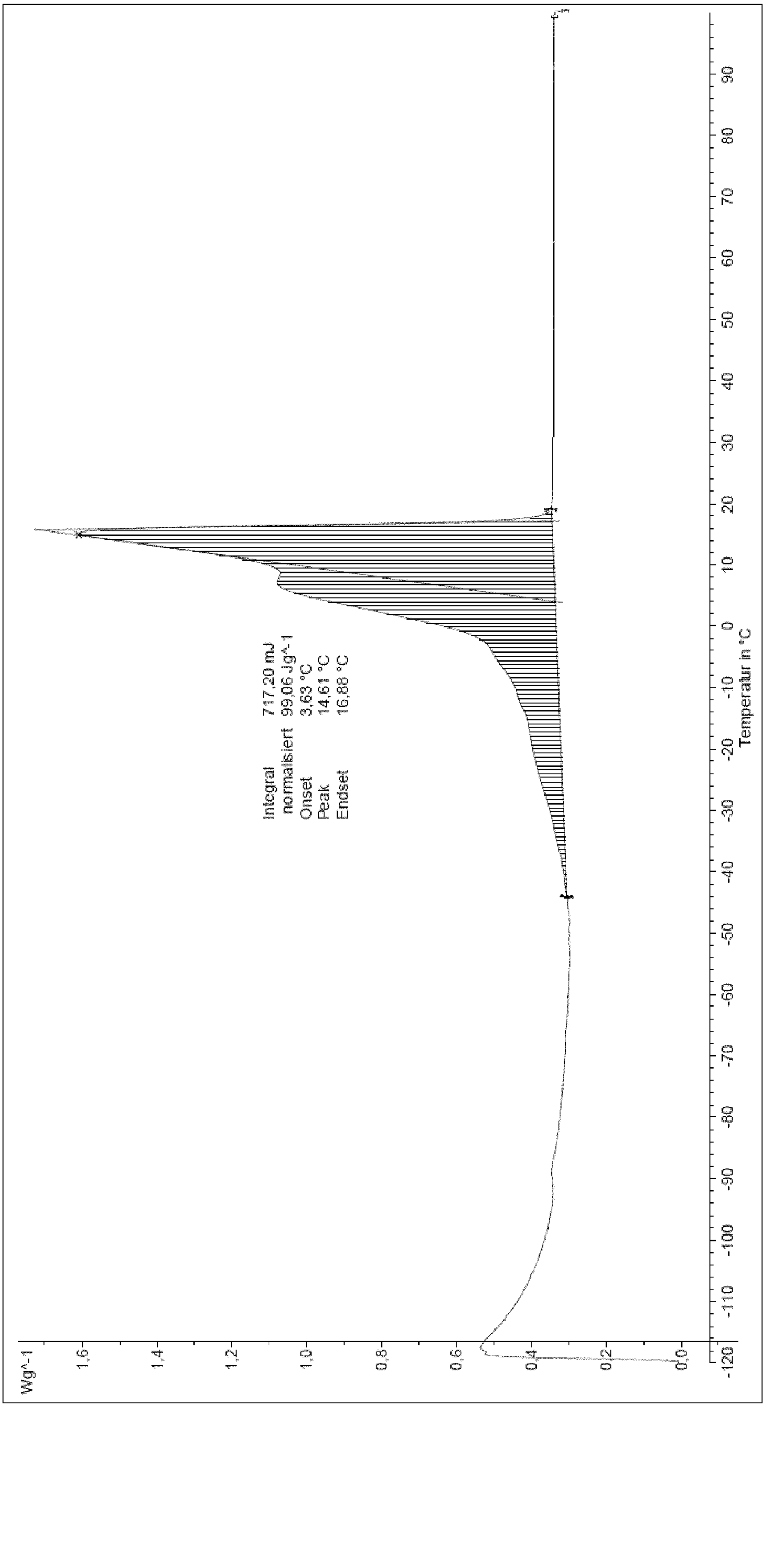


Abbildung 7: DSC von DPT (77%n, ↑endo)

Abbildung 8: DSC von DPT (100%n, ↑endo)

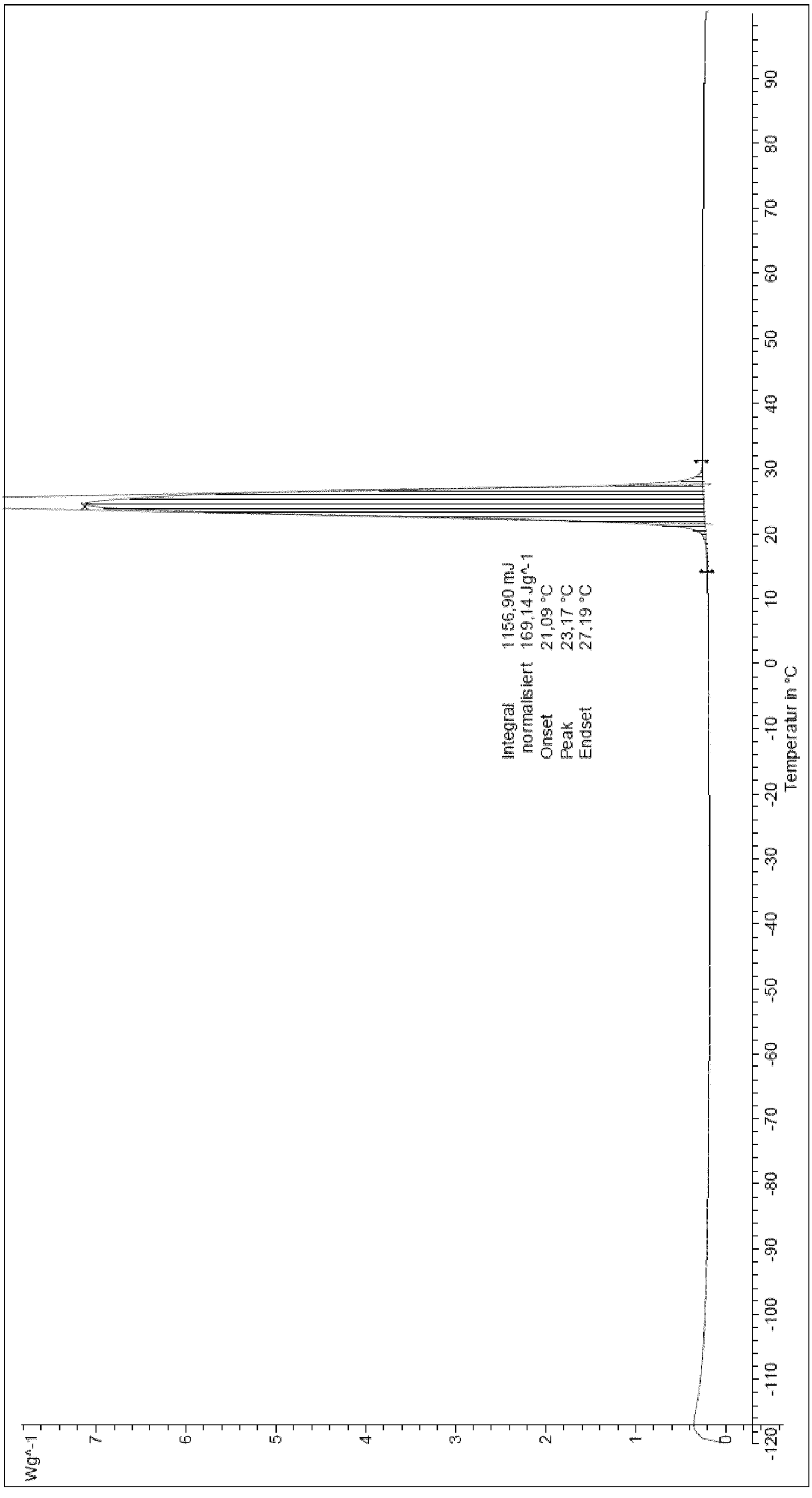

Abbildung 9: Plastisolviskosität nach 7-tägiger Lagerung bei 25 °C

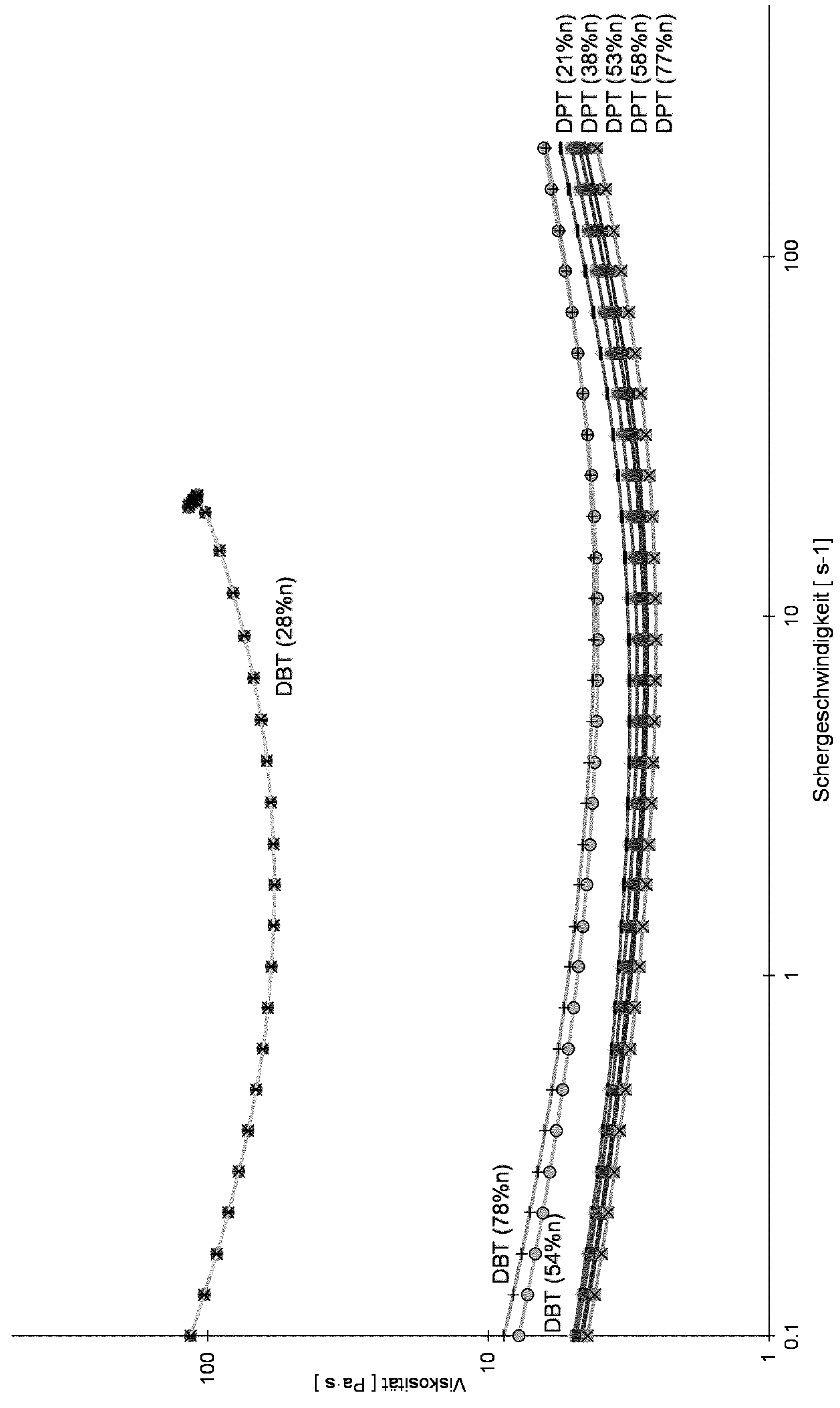

Abbildung 10: Eindicken der Plastisole

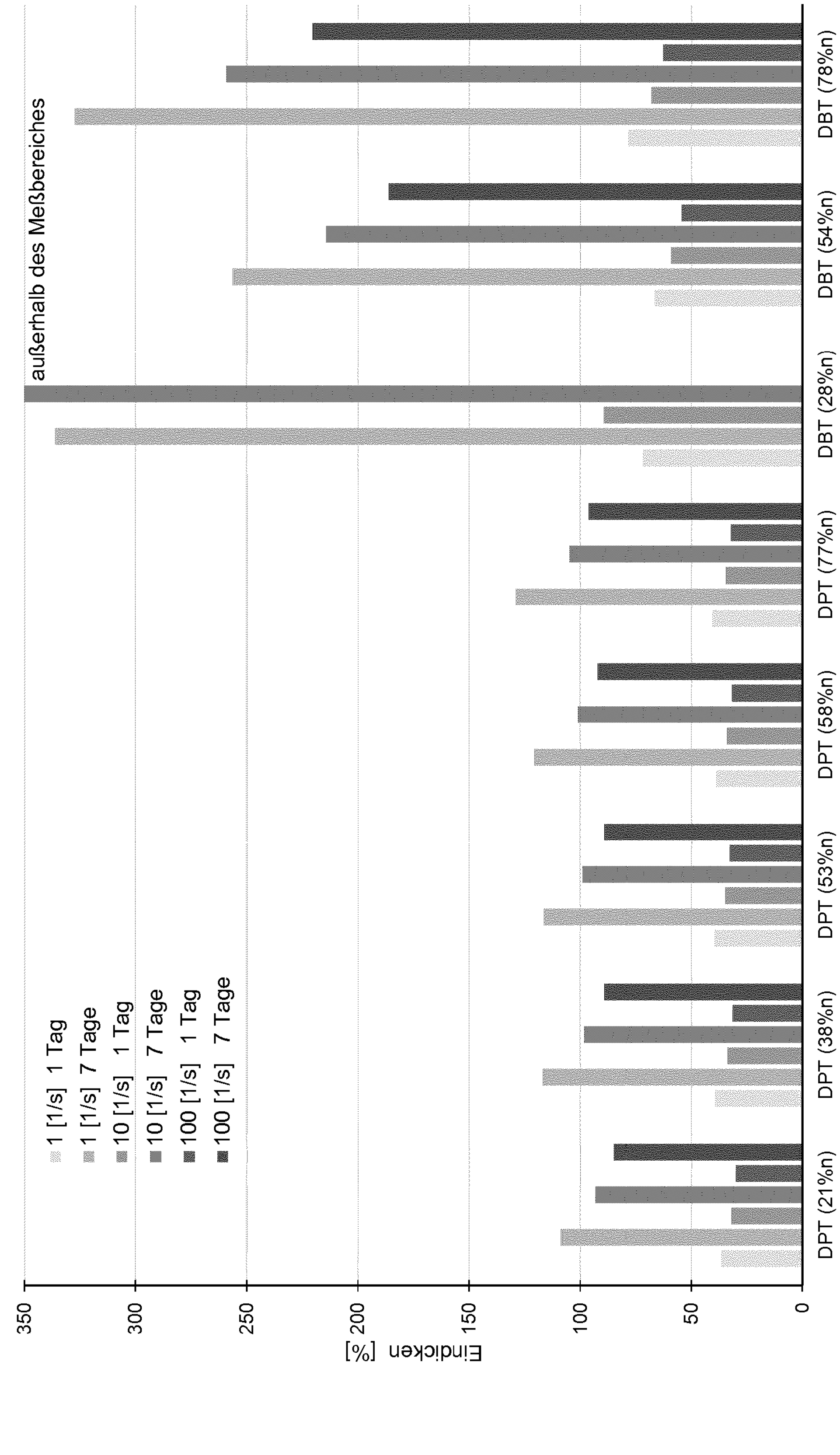

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1808457 B1
- WO 2010071717 A1